# EUROPÄISCHE PATENTSCHRIFT

(11) **EP 1 957 193 B1**
(45) Veröffentlichungstag und Bekanntmachung des Hinweises auf die Patenterteilung: **03.10.2012**
(21) Anmeldenummer: 06818628.7
(22) Anmeldetag: 17.11.2006
(51) Int. Cl.: B01J 20/26, A61L 15/22

(54) **GERUCHSBINDENDE SUPERABSORBIERENDE ZUSAMMENSETZUNG**
DEODORIZING SUPER-ABSORBENT COMPOSITION
COMPOSITION SUPERABSORBANTE RETENANT LES ODEURS

(30) Priorität: 18.11.2005 DE 102005055497
(43) Veröffentlichungstag der Anmeldung: 20.08.2008
(73) Patentinhaber: Evonik Stockhausen GmbH, 47805 Krefeld (DE)
(72) Erfinder: FURNO, Franck, 40545 Düsseldorf (DE); MÜLLER, Felix, 42555 Velbert (DE); PEGGAU, Jörg, 45357 Essen (DE); KEUP, Michael, 45139 Essen (DE); SCHMIDT, Harald, 47918 Tönisvorst (DE)
(86) Internationale Anmeldenummer: PCT/EP2006/011055
(87) Internationale Veröffentlichungsnummer: WO 2007/057203

(56) Entgegenhaltungen:
- EP-A- 1 358 894
- WO-A-01/70191
- US-A- 4 172 123
- US-A1- 2003 135 172
- US-A1- 2004 024 374

## Beschreibung

Die Erfindung betrifft im allgemeinen eine geruchsabsorbierende superabsorbierende Zusammensetzung, ein Verfahren zur Herstellung einer geruchsabsorbierenden superabsorbierenden Zusammensetzung, einen Verbund, beinhaltend eine geruchsabsorbierende superabsorbierende Zusammensetzung, einen Hygieneartikel, beinhaltend einen Verbund, chemische Produkte beinhaltend oder basierend auf einer geruchsabsorbierenden superabsorbierenden Zusammensetzung oder einem Verbund sowie die Verwendung einer geruchsabsorbierenden superabsorbierenden Zusammensetzung oder eines Verbunds in chemischen Produkten.

Superabsorber sind wasserunlösliche, vernetzte Polymere, die in der Lage sind, unter Quellung und Ausbildung von Hydrogelen größe Mengen an wässrigen Flüssigkeiten, insbesondere Körperflüssigkeiten, vorzugsweise Urin oder Blut, aufzunehmen und unter Druck zurückzuhalten. Im Allgemeinen betragen diese Flüssigkeitsaufnahmen das mindestens 10-Fache oder gar das mindestens 100-Fache des Trockengewichts der Superabsorber bzw. der superabsorbierenden Zusammensetzungen an Wasser. Durch diese charakteristischen Eigenschaften finden diese Polymere hauptsächlich Anwendung in Sanitärartikeln wie Babywindeln, Inkontinenzprodukten oder Damenbinden. Einen umfassenden Überblick über Superabsorber bzw. superabsorbierende Zusammensetzungen, ihre Anwendung und ihre Herstellung geben F. L. Buchholz und A. T. Graham (Herausgeber) in "Modem Superabsorbent Polymer Technology", Wiley-VCH, New York, 1998.

Die Herstellung der Superabsorber erfolgt in der Regel durch die radikalische Polymerisation säuregruppen-tragender, meist teilneutralisierter Monomere in Gegenwart von Vernetzern. Dabei lassen sich durch die Auswahl der Monomerzusammensetzung, der Vernetzer sowie der Polymerisationsbedingungen und der Verarbeitungsbedingungen für das nach der Polymerisation erhaltene Hydrogel Polymere mit unterschiedlichen Absorbereigenschaften herstellen. Weitere Möglichkeiten bietet die Herstellung von Pfropfpolymerisaten, beispielsweise unter Verwendung chemisch modifizierter Stärke, Cellulose und Polyvinylalkohol nach DE-OS 26 12 846.

DE 40 20 780 C 1 offenbart die Nachbehandlung superabsorbierender Polymere durch Nachvernetzung der Oberflächen der Polymerteilchen. Durch die Nachvernetzung der Oberfläche der wasserabsorbierenden Polymerteilchen wird insbesondere das Absorptionsvermögen der Polymerteilchen unter der Einwirkung von Drücken erhöht.

DE 199 09 653 A1 und DE 199 09 838 A1 beschreiben pulverförmige, an der Oberfläche nachvemetzte, Wasser, wässrige oder seröse Flüssigkeiten oder Blut absorbierende Polymerisate, welche auf säuregruppen-tragenden Monomeren basieren und welche mit einem Oberflächennachvernetzungsmittel und einem Kation in wässriger Lösung beschichtet und nachvernetzt worden sind. Die in diesem Stand der Technik offenbarten Polymerisate weisen gegenüber herkömmlichen Polymerisaten vorteilhafte Absorptionseigenschaften, insbesondere eine hohe Permeabilität auf.

Beim längeren Tragen von absorbierende Polymere beinhaltenden Hygieneartikeln, insbesondere wenn diese bereits zu einem Teil Körperflüssigkeiten wie Urin aufgenommen haben, kann es bedingt durch die organischen Bestandteile des Urins und die Körperwärme der Trägerperson alsbald zu einer unangenehmen Geruchsbelastung kommen. Um dieser zu begegnen, wurden zahlreiche Versuche unternommen, durch entsprechende Beigaben in den vom Superabsorber verschiedenen Bestandteilen des Hygieneartikels eine Bindung der geruchsbildenden Stoffe zu erzielen oder den durch diese gebildeten Geruch durch Parfums oder dergleichen zu übertönen. Das Einbringen von derartigen Substanzen in der Form von Superabsorber verschiedenen Bestandteilen wirkt sich oftmals negativ auf die Performance dieser Hygieneartikel während des Tragens aus. So werden durch die von Superabsorber verschiedenen Bestandteilen wirkt sich oftmals negativ auf die Performance dieser Hygieneartikel während des Tragens aus. So werden durch die Körperflüssigkeiten oftmals die geruchshemmenden oder -vermindernden Substanzen aus den vom Superabsorber verschiedenen Bestandteilen in den Superabsorber wie durch Hineinschwemmen übertragen wird, wo diese eine negative Auswirkung auf die Performance des Superabsorbers und damit des Hygieneartikels insgesamt haben. Ferner ist an diesem Konzept nachteilig, dass der Großteil der in den Hygieneartikel abgegebenen Körperflüssigkeit sich ohnehin in dem Superabsorber befindet und daher die außerhalb des Superabsorbers befindlichen geruchshemmenden oder geruchsvermindernden Substanzen ihre Wirkung schlechter entfalten können.

DE 198 25 486 und DE 199 39 662 A1 offenbaren die Kombination von Superabsorbern mit Cyclodextrin zur Geruchsverminderung. Diesem durchaus vielversprechenden Ansatz ist jedoch zu entnehmen, dass das Cyclodextrin nur unter bestimmten Bedingungen, nämlich wenn sichergestellt ist, dass das Cyclodextrin nicht von dem Superabsorber wieder separiert, seine geruchshemmende Wirkung in dem Superabsorber zeigt. Hierbei ist es bevorzugt, dass das Cyclodextrin zumindest in die Oberfläche des Superabsorberartikels eingearbeitet ist, indem Cyclodextrin und/oder Cyclodextrinderivate kovalent und/oder ionisch gebunden und/oder darin eingeschlossen sind.

Die US 2003/0135172 offenbart die Verwendung von Chelatliganden, wie EDTA, als geruchsbindende Mittel. Ebenfalls offenbart die EP1 358 896 die Verwendung von chelatisierenden Aminocarbonsäuren.

Im Allgemeinen lag der vorliegenden Erfindung die Aufgabe zugrunde, die sich aus dem Stand der Technik ergebenden Nachteile abzumildern oder gar zu überwinden.

Eine erfindungsgemäße Aufgabe bestand darin, eine superabsorbierende Zusammensetzung bereitzustellen, die zum einen über gute Geruchsbindungseigenschaften verfügt. Dabei soll zum anderen gewährleistet sein, dass die Performance des Im Allgemeinen lag der vorliegenden Erfindung die Aufgabe zugrunde, die sich aus dem Stand der Technik ergebenden Nachteile abzumildern oder gar zu überwinden.

Eine erfindungsgemäße Aufgabe bestand darin, eine superabsorbierende Zusammensetzung bereitzustellen, die zum einen über gute Geruchsbindungseigenschaften verfügt. Dabei soll zum anderen gewährleistet sein, dass die Performance des Hygieneartikels, der diese geruchsbindende Superabsorberzusammensetzung beinhaltet, im wesentlichen gleich gut oder gar besser ist als die Performance des Hygieneartikels mit einem Superabsorber, der nicht wie die geruchsbindende superabsorbierende Zusammensetzung den Geruchsbinder beinhaltet, zeigt.

Ferner bestand eine erfindungsgemäße Aufgabe darin, ein Verfahren aufzuzeigen, um eine solche superabsorbierende Zusammensetzung zu erhalten.

Zudem bestand eine erfindungsgemäße Aufgabe darin, einen Hygieneartikel bereitzustellen, der neben guten geruchsbindenden Eigenschaften auch eine gute Performance zeigt. In diesem Zusammenhang von besonderer Bedeutung ist die Aufnahmegeschwindigkeit des Hygieneartikels bei mehrfachem Einnässen und die Flüssigkeitsverteilung. Beide Eigenschaften sind im Zusammenhang mit einem guten Tragekomfort des Hygieneartikels neben der Geruchsreduzierung von Bedeutung.

Außerdem lag der vorliegenden Erfindung als Aufgabe zugrunde, superabsorbierende geruchsbindende Substanzen bereitzustellen, die sich allgemein in Verbunde einarbeiten lassen oder auch als Verbund oder als solche Verwendung in chemischen Produkten oder deren Bestandteile finden können.

Einen Beitrag zur Lösung der erfindungsgemäßen Aufgaben leisten die in den Haupt- und Nebenansprüchen umrissenen Gegenstände, wobei die Unteransprüche bevorzugte Ausführungsformen darstellen.

So betrifft die Erfindung in einer Ausgestaltung eine geruchsabsorbierende superabsorbierende Zusammensetzung, beinhaltend
i. ein wasserabsorbierendes Polymergebilde, worin das eingesetzte wasserabsorbierende Polymergebilde aus
   (α1) 20-99,999 Gew.%, bevorzugt 55-98,99 Gew.% und besonders bevorzugt 70-98,79 Gew.% polymerisierten, ethylenisch ungesättigten, säuregruppen-tragenden Monomeren oder deren Salze oder polymerisierten, ethylenisch ungesättigten, einen protonierten oder quarternierten Stickstoff beinhaltenden Monomeren, oder deren Mischungen, wobei mindestens ethylenisch ungesättigte, säuregruppenhaltige Monomere, vorzugsweise Acrylsäure, beinhaltende Mischungen besonders bevorzugt sind,
   (α2) 0-80 Gew.%, vorzugsweise 0-44,99 Gew.% und besonders bevorzugt 0,1-44,89 Gew.% polymerisierten, monoethylenisch ungesättigten, mit (α1) copolymerisierbaren Monomeren,
   (α3) 0,001-5 Gew.-%, vorzugsweise 0,01-3 Gew.-% und besonders bevorzugt 0,01-2,5 Gew.% eines oder mehrerer Vernetzer,
   (α4) 0 bis 5 Gew.%, vorzugsweise 0,001 bis 2,5 Gew.% und am meisten bevorzugt 0,01 bis 1 Gew.% des Metallsalzes,
   (α5) 0 bis 5 Gew.%, vorzugsweise 0,01 bis 2,5 Gew.% und am meisten bevorzugt 0,1 bis 1 Gew.% des Oxides des Metalls,
   (a6) 0 bis 30 Gew.-%, vorzugsweise 0 bis 5 Gew.% und besonders bevorzugt 0,1-5 Gew.% eines wasserlöslichen Polymeren,
   (α7) 0 bis 20 Gew.%, vorzugsweise 2,5 bis 15 Gew.% und besonders bevorzugt 3 bis 10 Gew.% Wasser, sowie (α8) 0 bis 20 Gew.-%, vorzugsweise 0 bis 10 Gew.-% und besonders bevorzugt 0,1 bis 8 Gew.% eines oder mehrerer Hilfsmittel, wobei die Summe der Gewichtsmengen (α1) bis (α8) 100 Gew.-% bestehen;
i. einen Geruchsbinder beinhaltend als Komponenten
   b1. eine Aminosäure; oder
   b2. eine Verbindung der allgemeinen Fromel I 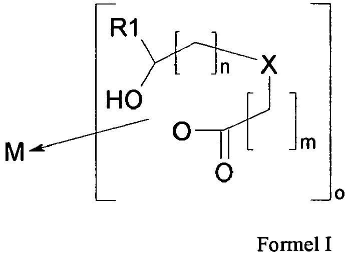
mit
- R1: als einen C₁-C₂₀-Kohlenwasserstoff;
- X: als nicht vorhanden oder ein in einem Bereich von 1 bis 5, vorzugsweise in einem Bereich von 1 bis 3 Doppelbindungen aufweisendes konjugiertes Doppelbindungssystem oder besonders bevorzugt eine einzige Doppelbindung;
- M: als ein geladenes oder ungeladenes, vorzugsweise geladenes Metall;
- m: in einem Bereich von 1 bis 15;
- n: in einem Bereich von 1 bis 5;
- o: in einem Bereich von 1 bis 4,

Aus der Darstellung von Formel I ist weiterhin zu erkennen, dass es bevorzugt ist, dass das geladene oder ungeladene, vorzugsweise geladene Metall M an einem Sauerstoff der Carboxylgruppe oder einem Sauerstoff der OH-Gruppe oder beiden koordiniert ist. Vorzugsweise liegt der durch []ₒ eingerahmte Teil der Formel I als Anion vor, der mindestens einen Teil der Ladung des als Kation vorliegenden Metalls kompensiert. Die Variablen m, n und o bilden Bereiche, in die alle denkbaren Zahlen einschließen, wobei die Zahlen vorzugsweise ganze natürliche Zahlen sind.

Als Kohlenwasserstoffe für den Rest R1 kommen alle dem Fachmann bekannten und geeigneten in Betracht. Hierunter fallen aliphatische, Doppelbindungen aufweisende oder aromatische Kohlenwasserstoffe oder Acrylalkyle. Die bevorzugten aliphatischen oder Doppelbindungen aufweisenden Kohlenwasserstoffe können sowohl cyklisch, verzweigt als auch unverzweigt, vorzugsweise unverzweigt sein.

In einer weiteren Ausgestaltung der erfindungsgemäßen superabsorbierenden Zusammensetzung ist es bevorzugt, dass der Geruchsbinder in einer Menge in einem Bereich von 0,01 bis 20 Gew.-%, vorzugsweise in einem Bereich von 0,1 bis 10 Gew.-% und darüber hinaus bevorzugt in einem Bereich von 1 bis 7 Gew.-%, jeweils bezogen auf das wasserabsorbierende Polymergebilde, in der superabsorbierenden Zusammensetzung enthalten ist.

Es entspricht einer anderen Ausgestaltung der erfindungsgemäßen superabsorbierenden Zusammensetzung, dass der Geruchsbinder die Komponenten b1 und b2 gleichzeitig beinhaltet. In diesem Fall ist es bevorzugt, dass die Komponente b1 in einer Menge in einem Bereich von 0,001 bis 20 Gew.-%, vorzugsweise in einem Bereich von 0,1 bis 10 Gew.-% und darüber hinaus bevorzugt in einem Bereich von 1 bis 7 Gew.-%, jeweils bezogen auf das wasserabsorbierende Polymergebilde, in der erfindungsgemäßen superabsorbierenden Zusammensetzung enthalten ist. Außerdem entspricht es einer Ausgestaltung der erfindungsgemäßen superabsorbierenden Zusammensetzung, bei der die Komponente b2 in einer Menge in einem Bereich von 0,01 bis 20 Gew.-%, vorzugsweise in einem Bereich von 0,1 bis 10 Gew.-% und darüber hinaus bevorzugt in einem Bereich von 1 bis 7 Gew.-%, jeweils bezogen auf das wasserabsorbierende Polymergebilde, in der erfindungsgemäßen superabsorbierenden Zusammensetzung enthalten ist.

In einer anderen Ausgestaltung der erfindungsgemäßen superabsorbierenden Zusammensetzung ist es bevorzugt, dass das Metall M ausgewählt ist aus der Gruppe bestehend aus einwertigen, zweiwertigen und dreiwertigen Metalle, wobei die zweiwertigen aus gründen der besseren Reaktivität mit den zumeist nukleophilen Geruchsmolekülen bevorzugt werden. Dieses können aus chemischer Sicht Ti, V, Mn, Fe, Co, Ni, Zn, Cd und Hg sein. Jedoch wird aus toxikologischen und ökologischen Gründen Zn besonders bevorzugt, das sich auch für die Verwendung der Komponente b2 in einer superabsorbierenden Zusammensetzung oder in Hygieneartikel besonders eignet.

In der erfindungsgemäßen superabsorbierenden Zusammensetzung ist es besonders bevorzugt, wenn die Komponente b2 ein Metallsalz einer unverzweigten oder verzweigten, ungesättigten oder gesättigten, ein oder mehrfach hydroxylierten Fettsäure mit mindestens 16 und oftmals bis zu 100 Kohlenstoffatomen oder deren Salz sowie aus beliebigen Mischungen von mindestens zwei dieser Fettsäuren oder deren Salze ist. Insbesondere können Zinksalze in Kombinationen mit Zinksalzen der Harzsäuren, insbesondere Abietinsäure, oder mit Zinksalzen anderer gesättigter oder ungesättigter hydroxylierter Fettsäuren mit 16 und oftmals bis zu 100 Kohlenstoffatomen eingesetzt werden, wobei insbesondere sind Zinksalze von Fettsäuren, vorzugsweise der Ricinolsäure, deren Herstellung in der DE-B-17 92 074 beschrieben ist, allein oder in Kombination mit anderen Wirkstoffen und/oder bekannte Geruchsabsorber, besonders bevorzugt sind. Als Komponenten b1 darüber hinaus bevorzugt ist Zinksalz der Ricinolsäure.

Als Aminosäure in der Komponente b1 kommt grundsätzlich jede dem Fachmann im Zusammenhang mit der vorliegenden Erfindung sinnvoll erscheinende Aminosäure in Betracht. Unter den Begriff Aminosäure fallen vorliegend sowohl die jeweiligen Aminosäuren, deren Derivate als auch die Salze dieser Aminosäuren bzw. dieser Derivate. In einer erfindungsgemäßen Ausgestaltung werden zwei verschiedene Aminosäuren in der Komponente b1 kombiniert. Unter den Aminosäuren sind insbesondere α-, β- oder γ-Aminosäuren bevorzugt, wobei α-Aminosäuren besonders bevorzugt sind. So können beispielsweise zwei α-Aminosäuren kombiniert werden. Unter den α-Aminosäuren sind besonders die Aminosäuren Methionin, Arginin und Cystein bevorzugt, wobei Cystein und Arginin besonders bevorzugt sind. Es kann jedoch neben dem Cystein oder auch für sich die Aminosäure Arginin als solche eingesetzt werden oder zumindest die Aminosäure mit der höchsten Konzentration in dem Geruchsbinder darstellen. Dieses gilt insbesondere für den Fall, dass die Komponenten b1 und b2 gleichzeitig eingesetzt werden. Bei Aminosäuren beinhaltenden Geruchsbinder sollte darauf geachtet werden, dass die darin enthaltenen Aminosäuren ihre geruchsbindende Wirkung nicht durch eine zu starke thermische Belastung bei der Herstellung oder Weiterverarbeitung der erfindungsgemäßen geruchsabsorbierenden superabsorbierenden Zusammensetzung erfahren.

Nach einer anderen Ausgestaltung der erfindungsgemäßen superabsorbierenden Zusammensetzung ist es bevorzugt, dass diese weiterhin iii. einen von dem wasserabsorbierenden Polymergebilde verschiedenen polymeren Binder beinhaltet. Als polymere Binder kommen grundsätzlich alle dem Fachmann für den Zweck der Fixierung des Geruchsbinders bekannten und geeignet erscheinenden polymeren Binder in Betracht. Unter diesen sind thermoplastische Polymere bevorzugt.

In diesem Zusammenhang wird auf die in WO 2005/011860 beschriebenen thermoplastischen Polymere Bezug genommen. Vorzugsweise weisen derartige polymere Binder ein durch GPC (Gelpermeationschromatographie) bestimmtes und mittels Lichtstreuung absolut detektierbares Molekulargewicht in einem Bereich von 1000 bis 100.000, vorzugsweise 2000 bis 50.000 und darüber hinaus bevorzugt 3000 bis 15.000 g/Mol auf. Weiterhin ist es bevorzugt, dass derartige polymere Binder zwei oder mehr OH-Gruppen beinhalten. Beispiele geeigneter polymerer Binder sind Polyalkylenglykole, wie Polyethylenglykol, Polypropylenglykol, wobei Polyethylenglykol und insbesondere ein Polyethylenglykol mit einem Molekulargewicht in einem Bereich von 5000 bis 15.000 g/mol, besonders bevorzugt ist. Die hier angegebenen Molekulargewichte betreffen jeweils das Gewichtsmittel des Molekulargewichts.

Erfindungsgemäß bevorzugte Polymergebilde sind Fasern, Schäume oder Teilchen, wobei Fasern und Teilchen bevorzugt und Teilchen besonders bevorzugt sind.

In einer Ausgestaltung der vorliegenden Erfindung ist es bevorzugt, dass in der erfindungsgemäße Zusammensetzung das wasserabsorbierende Polymergebilde auf weniger als 40 Gew.%, vorzugsweise weniger als 30 Gew.-%, besonders bevorzugt weniger als 20 Gew.-% und darüber hinaus bevorzugt weniger als 10 Gew.%, jeweils bezogen auf das wasserabsorbierende Polymergebilde, Superabsorberfeinteilchen einer Partikelgröße von weniger als 150 µm, bestimmt nach ERT 420.1-99, basiert. Die Superabsorberfeinteilchen können einen gleichen jedoch auch von den wasserabsorbierenden Polymergebilden verschiedenen chemischen Aufbau besitzen. Im Allgemeinen sind sich die Superabsorberfeinteilchen und die in der erfindungsgemäßen Zusammensetzung enthaltenen, wasserabsorbierenden Polymergebilde in ihrem chemischen Aufbau ähnlich und unterscheiden sich vornehmlich durch die Teilchengröße.

Einem weiteren Aspekt der erfindungsgemäßen Zusammensetzung nach kann das wasserabsorbierende Polymergebilde mindestens 60 Gew.-%, vorzugsweise mindestens 70 Gew.-%, besonders bevorzugt mindestens 80 Gew.-% und darüber hinaus bevorzugt mindestens 90 Gew.-%, jeweils bezogen auf das wasserabsorbierende Polymergebilde, Teilchen mit einer Teilchengröße in einem Bereich von 150 bis 850 µm, bestimmt nach ERT 420.1-99, aufweisen.

In einer weiteren Ausgestaltung der erfindungsgemäßen Zusammensetzung basiert diese auf weniger als 20 Gew.-%, vorzugsweise weniger als 10 Gew.-% und besonders bevorzugt weniger als 5 Gew.%, jeweils bezogen auf die Zusammensetzung, auf einem vernetzbaren, nicht vernetzten Polymer. Im Zusammenhang mit dem vernetzbaren, nicht vernetzten Polymer wird als Teil des vorliegenden Texts auf DE 103 34 271 A1 und dort insbesondere auf die Ausführungen in den Abschnitten [0063] bis [0082] verwiesen.

Erfindungsgemäß bevorzugte Polymerfasern sind so dimensioniert, dass sie in oder als Garne für Textilien und auch direkt in Textilien eingearbeitet werden können. Es ist erfindungsgemäß bevorzugt, dass die als Polymerfasern vorliegenden Polymergebilde eine Länge in einem Bereich von 1 bis 500 mm, bevorzugt 2 bis 500 mm und besonders bevorzugt 5 bis 100 mm und einen Durchmesser in einem Bereich von 1 bis 200 Denier, bevorzugt 3 bis 100 Denier und besonders bevorzugt 5 bis 60 Denier besitzen.

Erfindungsgemäß bevorzugte Polymerteilchen sind so dimensioniert, dass sie eine mittlere Teilchengröße gemäß ERT 420.2-02 in einem Bereich von 10 bis 3000 µm, vorzugsweise 20 bis 2000 µm und besonders bevorzugt 150 bis 850 µm aufweisen. Dabei ist es insbesondere bevorzugt, dass der Anteil der Polymerteilchen mit einer Partikelgröße in einem Bereich von 300 bis 600 µm mindestens 30 Gew.-%, besonders bevorzugt mindestens 40 Gew.-% und am meisten bevorzugt mindestens 50 Gew.%, bezogen auf das Gesamtgewicht der nachvernetzten, wasserabsorbierenden Polymerteilchen, beträgt.

In der Ausführungsform der erfindungsgemäß eingesetzten wasserabsorbierenden Polymergebilde basieren diese auf
- (α1): 20-99,999 Gew.-%, bevorzugt 55-98,99 Gew.-% und besonders bevorzugt 70-98,79 Gew.-% polymerisierten, ethylenisch ungesättigten, säuregruppen-tragenden Monomeren oder deren Salze oder polymerisierten, ethylenisch ungesättigten, einen protonierten oder quarternierten Stickstoff beinhaltenden Monomeren, oder deren Mischungen, wobei mindestens ethylenisch ungesättigte, säuregruppenhaltige Monomere, vorzugsweise Acrylsäure, beinhaltende Mischungen besonders bevorzugt sind,
- (α2): 0-80 Gew.-%, vorzugsweise 0-44,99 Gew.-% und besonders bevorzugt 0,1-44,89 Gew.% polymerisierten, monoethylenisch ungesättigten, mit (α1) copolymerisierbaren Monomeren,
- (α3): 0,001-5 Gew.-%, vorzugsweise 0,01-3 Gew.-% und besonders bevorzugt 0,01-2,5 Gew.-% eines oder mehrerer vernetzer,
- (α4): 0 bis 5 Gew.%, vorzugsweise 0,001 bis 2,5 Gew.% und am meisten bevorzugt 0,01 bis 1 Gew.-% des Metallsalzes,
- (α5): 0 bis 5 Gew.-%, vorzugsweise 0,01 bis 2,5 Gew.-% und am meisten bevorzugt 0,1 bis 1 Gew.% des Oxides des Metalls,
- (α6): 0 bis 30 Gew.-%, vorzugsweise 0 bis 5 Gew.-% und besonders bevorzugt 0,1-5 Gew.-% eines wasserlöslichen Polymeren,
- (α7): 0 bis 20 Gew.%, vorzugsweise 2,5 bis 15 Gew.-% und besonders bevorzugt 3 bis 10 Gew.-% Wasser, sowie
- (α8): 0 bis 20 Gew.-%, vorzugsweise 0 bis 10 Gew.-% und besonders bevorzugt 0,1 bis 8 Gew.% eines oder mehrerer Hilfsmittel, wobei die Summe der Gewichtsmengen (α1) bis (α8) 100 Gew.% beträgt.

Die monoethylenisch ungesättigten, säuregruppenhaltigen Monomere (α1) können teilweise oder vollständig, bevorzugt teilweise neutralisiert sein. Vorzugsweise sind die monoethylenisch ungesättigten, säuregruppenhaltigen Monomere zu mindestens 25 Mol-%, besonders bevorzugt zu mindestens 50 Mol-% und darüber hinaus bevorzugt zu 50-80 Mol-% neutralisiert. In diesem Zusammenhang wird auf DE 195 29 348 A1 verwiesen, deren Offenbarung hiermit als Referenz eingeführt wird. Die Neutralisation kann teilweise oder ganz auch nach der Polymerisation erfolgen. Ferner kann die Neutralisation mit Alkalimetallhydroxiden, Erdalkalimetallhydroxiden, Ammoniak sowie Carbonaten und Bicarbonaten erfolgen. Daneben ist jede weitere Base denkbar, die mit der Säure ein wasserlösliches Salz bildet. Auch eine Mischneutralisation mit verschiedenen Basen ist denkbar. Bevorzugt ist die Neutralisation mit Ammoniak und Alkalimetallhydroxiden, besonders bevorzugt mit Natriumhydroxid und mit Ammoniak.

Ferner können bei einem Polymer die freien Säuregruppen überwiegen, so dass dieses Polymer einen im sauren Bereich liegenden pH-Wert aufweist. Dieses saure wasserabsorbierende Polymer kann durch ein Polymer mit freien basischen Gruppen, vorzugsweise Amingruppen, das im Vergleich zu dem sauren Polymer basisch ist, mindestens teilweise neutralisiert werden. Diese Polymere werden in der Literatur als "Mixed-Bed Ion-Exchange Absorbent Polymers" (MBIEA-Polymere) bezeichnet und sind unter anderem in der WO 99/34843 A1 offenbart. Die Offenbarung der WO 99/34843 A1 wird hiermit als Referenz eingeführt und gilt somit als Teil der Offenbarung. In der Regel stellen MBIEA-Polymere eine Zusammensetzung dar, die zum einen basische Polymere, die in der Lage sind, Anionen auszutauschen, und andererseits ein im Vergleich zu dem basischen Polymer saures Polymer, das in der Lage ist, Kationen auszutauschen, beinhalten. Das basische Polymer weist basische Gruppen auf und wird typischerweise durch die Polymerisation von Monomeren erhalten, die basische Gruppen oder Gruppen tragen, die in basische Gruppen umgewandelt werden können. Bei diesen Monomeren handelt es sich vor allen Dingen um solche, die primäre, sekundäre oder tertiäre Amine oder die entsprechenden Phosphine oder mindestens zwei der vorstehenden funktionellen Gruppen aufweisen. Zu dieser Gruppe von Monomeren gehören insbesondere Ethylenamin, Allylamin, Diallylamin, 4-Aminobuten, Alkyloxycycline, Vinylformamid, 5-Aminopenten, Carbodiimid, Formaldacin, Melamin und dergleichen, sowie deren sekundäre oder tertiäre Aminderivate. Bevorzugte ethylenisch ungesättigte, säuregruppenhaltige Monomere (α1) sind vorzugsweise diejenigen Verbindungen, die in der WO 2004/037903 A2, die hiermit als Referenz eingeführt wird und somit als Teil der Offenbarung gilt, als ethylenisch ungesättigte, säuregruppenhaltige Monomere (α1) genannt werden. Besonders bevorzugte ethylenisch ungesättigte, säuregruppenhaltige Monomere (α1) sind Acrylsäure und Methacrylsäure, wobei Acrylsäure am meisten bevorzugt ist.

Gemäß einer Ausführungsform der vorliegenden Erfindung werden wasserabsorbierende Polymergebilde eingesetzt, bei denen die monoethylenisch ungesättigten, mit (α1) copolymerisierbare Monomere (α2) Acrylamid, Methacrylamide oder Vinylamide sind.

Bevorzugte (Meth)acrylamide sind neben Acrylamid und Methacrylamid alkylsubstituierte (Meth)acrylamide oder aminoalkylsubstituierte Derivate des (Meth)acrylamids, wie N-Methylol(meth)acrylamid, N,N-Dimethylamino-(meth)acrylamid, Dimethyl(meth)acrylamid oder Diethyl(meth)acrylamid. Mögliche Vinylamide sind beispielsweise N-Vinylamide, N-Vinylformamide, N-Vinylacetamide, N-Vinyl-N-Methylacetamide, N-Vinyl-N-methylformamide, Vinylpyrrolidon. Unter diesen Monomeren besonders bevorzugt ist Acrylamid.

Gemäß einer anderen Ausführungsform der vorliegenden Erfindung werden wasserabsorbierende Polymergebilde eingesetzt, bei denen die monoethylenisch ungesättigten, mit (α1) copolymerisierbaren Monomere (α2) wasserlösliche Monomere sind. In diesem Zusammenhang sind insbesondere Alkoxypolyalkylenoxid(meth)acrylate wie Methoxypolyethylenglykol(meth)acrylate bevorzugt.

Des Weiteren sind als monoethylenisch ungesättigte, mit (α1) copolymerisierbaren Monomere (α2) in Wasser dispergierbare Monomere bevorzugt. Als in Wasser dispergierbare Monomere sind Acrylsäureester und Methacrylsäureester, wie Methyl(meth)acrylat, Ethyl(meth)acrylat, Propyl(meth)acrylat oder Butyl(meth)-acrylat.

Die monoethylenisch ungesättigten, mit (α1) copolymerisierbaren Monomere (α2) umfassen weiterhin Methylpolyethylenglykolallylether, Vinylacetat, Styrol und Isobutylen.

Als Vernetzer (α3) werden vorzugsweise diejenigen Verbindungen eingesetzt, die in der WO 2004/037903 A2 als Vernetzer (α3) genannt werden. Unter diesen Vernetzern sind wasserlösliche Vernetzer besonders bevorzugt. Am meisten bevorzugt sind dabei N,N'-Methylenbisacrylamid, Polyethylenglykoldi(meth)acrylate, Triallylmethylammoniumchlorid, Tetraallylammoniumchlorid sowie mit 9 Mol Ethylenoxid pro Mol Acrylsäure hergestelltes Allylnonaethylenglykolacrylat besonders bevorzugt.

Als Metallsalz (α4) können alle dem Fachmann bekannten Metallsalze, die wasserunlöslich oder wasserlöslich sein können, eingesetzt werden, wobei jedoch wasserlösliche Metallsalze besonders bevorzugt sind. Unter "wasserlöslich" werden dabei vorzugsweise Metallsalze verstanden, von denen bei einer Temperatur von 25°C vorzugsweise mindestens 1 g, besonders bevorzugt mindestens 10 g, darüber hinaus bevorzugt mindestens 100 g und am meisten bevorzugt mindestens 500 g in einem Liter destilliertem Wasser löslich sind.

Unter den vorstehend genannten, vorzugsweise wasserlöslichen Metallsalzen sind insbesondere Sulfate, Sulfite, Sulfide, Chloride, Bromide, Iodide, Nitrate, Nitrite, Phosphate, Phosphite, Carbonate, Hydrogencarbonate, Hydroxide, Acetate, Lactate und Oxalate bevorzugt, wobei Sulfate am meisten bevorzugt sind.

Weiterhin ist es erfindungsgemäß bevorzugt, dass das Metallkation des Metallsalzes ein einwertiges, zweiwertiges oder ein dreiwertiges Metallkation ist, wobei dreiwertige Metallkationen am meisten bevorzugt sind. Als Metallkationen bevorzugt sind Na⁺, K⁺, Li⁺, Mg²⁺, Ca²⁺, Fe²⁺, F³⁺, Ag⁺, Cu⁺, Cu²⁺, Zn²⁺, sowie Al³⁺, wobei als Al³⁺ am meisten bevorzugt ist.

Folgende Metallsalze sind in den erfindungsgemäßen wasserabsorbierenden Polymergebilden vorzugsweise als Oberflächenbehandlungsmittel enthalten: AlCl₃ x 6H₂0, NaAl(SO₄)₂ x 12 H₂O, Al(NO₃)₃ x 9 H₂O, KAl(SO₄)₂ x 12 H₂O oderAl₂(SO₄)₃ x 14-18 H₂O sowie die entsprechenden wasserfreien Salze, Na₂SO₄ oder dessen Hydrate, MgSO₄ x 10 H₂O oder wasserfreies Magnesiumsulfat.

Als wasserabsorbierende Polymergebilde werden erfindungsgemäße, vorzugsweise Polymere eingesetzt, welche erhalten wurden durch ein Verfahren umfassend die Verfahrensschritte:
a) radikalische Polymerisation säuregruppen-tragender, ethylenisch ungesättigter, gegebenenfalls teilneutralisierter Monomere in Gegenwart eines Vernetzers unter Bildung eines Hydrogels;
b) gegebenenfalls Zerkleinern des Hydrogels;
c) Trocknen des gegebenenfalls zerkleinerten Hydrogels unter Erhalt wasserabsorbierender Polymergebilde;
d) gegebenenfalls Zermahlen des so erhaltenen absorbierenden Polymergebildes und Absieben auf eine gewünschte Partikelgrößenfraktion;
e) gegebenenfalls weitere Oberflächenmodifizierungen der so erhaltenen wasserabsorbierenden Polymergebilde

Geeignete Mischaggregate zur Oberfächenmodifizierung und auch zum Aufbringen des Geruchsbinders oder zumindest einer seiner Komponenten sind der Patterson-Kelley-Mischer, DRAIS-Turbulenzmischer, Lödigemischer, Ruberg-Mischer, Schneckenmischer, Tellermischer und Wirbelschichtmischer sowie kontinuierlich arbeitende senkrechte Mischer, in denen das Polymergebilde mittels rotierender Messer in schneller Frequenz gemischt wird (Schugi-Mischer).

Die im Verfahrensschritt a) erfolgende radikalische Polymerisation erfolgt vorzugsweise in wässriger Lösung, wobei diese wässrige Lösung neben Wasser als Lösungsmittel vorzugsweise
- (α1): die ethylenisch ungesättigten, säuregruppen-tragenden Monomeren oder deren Salze, wobei Acrylsäure als säuregruppentragendes Monomer besonders bevorzugt ist,
- (α2): gegebenenfalls monoethylenisch ungesättigte, mit (α1) copolymerisierbare Monomere,
- (α3): den Vernetzer,
- (α6): gegebenenfalls ein wasserlösliches Polymer, sowie
- (α8): gegebenenfalls ein oder mehrere Hilfsmittel
beinhaltet.

Als ethylenisch ungesättigte, säuregruppen-tragenden Monomere (α1), als monoethylenisch ungesättigte, mit (α1) copolymerisierbare Monomere (α2), als Vernetzer (α3), als wasserlösliche Polymere (a6) und als Hilfsmittel (α8) sind wiederum diejenigen Verbindungen bevorzugt, die bereits eingangs im Zusammenhang mit den erfindungsgemäßen Polymergebilden als ethylenisch ungesättigte, säuregruppen-tragenden Monomere (α1), als monoethylenisch ungesättigte, mit (α1) copolymerisierbare Monomere (α2), als Vernetzer (α3), als wasserlösliche Polymere (α6) und als Hilfsmittel (α8) genannt wurden.

Aus den vorgenannten Monomeren, Comonomeren, Vernetzern, wasserlöslichen Polymeren und Hilfsstoffen lassen sich die wasserabsorbierende Polymergebilde durch verschiedene Polymerisationsweisen herstellen. Beispielsweise sind in diesem Zusammenhang Massepolymerisation, die vorzugsweise in Knetreaktoren wie Extrudern erfolgt, Lösungspolymerisation, Spraypolymerisation, inverse Emulsionspolymerisation und inverse Suspensionspolymerisation zu nennen.

Bevorzugt wird die Lösungspolymerisation in Wasser als Lösungsmittel durchgeführt. Die Lösungspolymerisation kann kontinuierlich durch Polymerisation auf einem die Reaktionsmischung weiterfördernden Band, wie in DE 35 44 770 A1 offenbart, oder diskontinuierlich erfolgen. Aus dem Stand der Technik ist ein breites Spektrum von Variationsmöglichkeiten hinsichtlich der Reaktionsverhältnisse wie Temperaturen, Art und Menge der Initiatoren als auch der Reaktionslösung zu entnehmen. Typische Verfahren sind in den folgenden Patentschriften beschrieben: US 4,286,082, DE 27 06 135, US 4,076,663, DE 35 03 458, DE 35 44 770, DE 40 20 780, DE 42 44 548, DE 43 23 001, DE 43 33 056, DE 44 18 818. Die Offenbarungen werden hiermit als Referenz eingeführt und gelten somit als Teil der Offenbarung.

Die Polymerisation wird wie allgemein üblich durch einen Initiator ausgelöst. Als Initiatoren zur Initiierung der Polymerisation können alle unter den Polymerisationsbedingungen Radikale bildende Initiatoren verwendet werden, die üblicherweise bei der Herstellung von Superabsorbern eingesetzt werden. Auch eine Initiierung der Polymerisation durch Einwirkung von Elektronenstrahlen auf die polymerisierbare, wässrige Mischung ist möglich. Die Polymerisation kann allerdings auch in Abwesenheit von Initiatoren der oben genannten Art durch Einwirkung energiereicher Strahlung in Gegenwart von Photoinitiatoren ausgelöst werden. Polymerisationsinitiatoren können in einer Lösung erfindungsgemäßer Monomere gelöst oder dispergiert enthalten sein. Als Initiatoren kommen sämtliche dem Fachmann bekannten in Radikale zerfallende Verbindungen in Betracht. Hierunter fallen insbesondere diejenigen Initiatoren, die bereits in der WO 2004/037903 A2 als mögliche Initiatoren genannt werden.

Besonders bevorzugt wird zur Herstellung der wasserabsorbierenden Polymergebilde ein Redoxsystem bestehend aus Wasserstoffperoxid, Natriumperoxodisulfat und Ascorbinsäure eingesetzt.

Auch die inverse Suspensions- und Emulsionspolymerisation kann zur Herstellung der Polymergebilde angewendet werden. Gemäß diesen Prozessen wird eine wässrige, teilneutralisierte Lösung der Monomere (α1), und (α2), gegebenenfalls beinhaltend wasserlösliche Polymere und Hilfsstoffe, mit Hilfe von Schutzkolloiden und/oder Emulgatoren in einem hydrophoben, organischen Lösungsmittel dispergiert und durch Radikalinitiatoren die Polymerisation gestartet. Die Vernetzer sind entweder in der Monomerlösung gelöst und werden mit dieser zusammen dosiert oder aber separat und gegebenenfalls während der Polymerisation zugefügt. Gegebenenfalls erfolgt die Zugabe eines wasserlöslichen Polymeren (a6) als Pfropfgrundlage über die Monomerlösung oder durch direkte Vorlage in die Ölphase. Anschließend wird das Wasser azeotrop aus dem Gemisch entfernt und das Polymerisat abfiltriert.

Weiterhin kann sowohl bei der Lösungspolymerisation als auch bei der inversen Suspensions- und Emulsionspolymerisation die Vernetzung durch Einpolymerisation des in der Monomerlösung gelösten polyfunktionellen Vernetzers und/oder durch Reaktion geeigneter Vernetzer mit funktionellen Gruppen des Polymeren während der Polymerisationsschritte erfolgen. Die Verfahren sind beispielsweise in den Veröffentlichungen US 4,340,706, DE 37 13 601, DE 28 40 010 und WO 96/05234 A1 beschrieben, deren entsprechende Offenbarung hiermit als Referenz eingeführt wird.

Die bei der Lösungspolymerisation oder der inversen Suspensions- und Emulsionspolymerisation im Verfahrensschritt a) erhaltenen Hydrogele werden im Verfahrensschritt c) getrocknet.

Insbesondere im Falle der Lösungspolymerisation ist es jedoch bevorzugt, dass die Hydrogele vor der Trocknung zunächst in einem zusätzlichen Verfahrensschritt b) zerkleinert werden. Dieses Zerkleinern erfolgt durch dem Fachmann bekannte Zerkleinerungsvorrichtungen, wie ein Hackmesser (vgl. DE 195 18 645 C1 oder etwa einen Fleischwolf, der dem Hackmesser nachgeschaltet sein kann.

Die Trocknung des Hydrogels erfolgt vorzugsweise in geeigneten Trocknern oder Öfen. Beispielhaft seien Drehrohröfen, Wirbelbetttrockner, Tellertrockner, Paddeltrockner oder Infrarottrockner genannt. Weiterhin ist es erfindungsgemäß bevorzugt, dass die Trocknung des Hydrogels im Verfahrensschritt c) bis zu einem Wassergehalt von 0,5 bis 25 Gew.-%, vorzugsweise von 1 bis 10 Gew.% erfolgt, wobei die Trocknungstemperaturen üblicherweise in einem Bereich von 100 bis 200°C liegen.

Die im Verfahrensschritt c) erhaltenen, getrockneten wasserabsorbierenden Polymergebilde können, insbesondere dann, wenn sie durch Lösungspolymerisation erhalten wurden, in einem weiteren Verfahrensschritt d) noch zermahlen und auf die eingangs genannte Wunschkomgröße abgesiebt werden. Das Zermahlen der getrockneten, wasserabsorbierenden Polymergebilde erfolgt vorzugsweise in geeigneten, mechanischen Zerkleinerungsvorrichtungen, wie etwa einer Kugelmühle.

Im Anschluss an die Trocknung der Hydrogele und nach der gegebenenfalls erfolgten weiteren Konfektionierung der getrockneten wasserabsorbierenden Polymergebilde können diese in einem weiteren Verfahrenschritt e) im Oberflächenbereich modifiziert werden. Dieses kann durch ein Metallsalz oder mit der Kombination aus dem Metallsalz und dem Oxid eines Metalls oder einem andere Modifizierungsmittel oder durch ein anderes Modifizierungsmittel erfolgen, wobei die vorstehenden Stoffe als wässrige Lösung oder als Feststoff aufgetragen werden können. Die Konfektionierung kann sowohl auf dem wasserabsorbierenden Polymergebilde als auch auf der den Geruchsbinder bereits beinhaltenden superabsorbierenden Zusammensetzung erfolgen.

Als bevorzugte Modifizierungsmaßnahme sei hier die Oberflächennachvernetzung genannt, bei der die getrockneten Polymergebilde oder aber das noch nicht getrocknete, jedoch vorzugsweise bereits zerkleinerte Hydrogel mit einem vorzugsweise organischen, chemischen Oberflächennachvernetzer in Kontakt gebracht wird. Dabei wird der Nachvernetzer insbesondere dann, wenn er unter den Nachvernetzungsbedingungen nicht flüssig ist, über ein Lösungsmittel mit den Polymerteilchen bzw. dem Hydrogel in Kontakt gebracht. Als Lösungsmittel werden dabei vorzugsweise Wasser, mit Wasser mischbare organische Lösungsmittel wie etwa Methanol, Ethanol, 1-Propanol, 2-Propanol oder 1-Butanol oder Mischungen aus mindestens zwei dieser Lösungsmittel eingesetzt, wobei Wasser als Lösungsmittel am meisten bevorzugt ist. Weiterhin ist es bevorzugt, dass der Nachvernetzer in dem Lösungsmittel oder -gemisch in einer Menge in einem Bereich von 5 bis 75 Gew.-%, besonders bevorzugt 10 bis 50 Gew.% und am meisten bevorzugt 15 bis 40 Gew.-%, bezogen auf das Gesamtgewicht des Lösungsmittels oder -gemisches, enthalten ist.

Das Inkontaktbringen des Polymergebildes bzw. des zerkleinerten Hydrogels mit dem Lösungsmittel oder -gemisch beinhaltend den Nachvernetzer erfolgt im erfindungsgemäßen Verfahren vorzugsweise durch gutes Vermischen des Lösungsmittels oder -gemischs mit dem Polymergebilde.

Geeignete Mischaggregate zum Vermischen sind z. B. der Patterson-Kelley-Mischer, DRAIS-Turbulenzmischer, Lödigemischer, Ruberg-Mischer, Schneckenmischer, Tellermischer und Wirbelschichtmischer sowie kontinuierlich arbeitende senkrechte Mischer, in denen das Polymergebilde mittels rotierender Messer in schneller Frequenz gemischt wird (Schugi-Mischer).

Das Polymergebilde wird in dem erfindungsgemäßen Verfahren bei der Nachvernetzung vorzugsweise mit höchstens 20 Gew. %, besonders bevorzugt mit höchstens 15 Gew.-%, darüber hinaus bevorzugt mit höchstens 10 Gew. -%, darüber hinaus noch mehr bevorzugt mit höchstens 5 Gew.-% an Lösungsmittel, vorzugsweise Wasser, in Kontakt gebracht.

Bei Polymergebilden in der Form von vorzugsweise näherungsweise kugelförmigen Teilchen ist es erfindungsgemäß weiterhin bevorzugt, dass das in Kontakt bringen derart erfolgt, dass lediglich der Außenbereich, nicht jedoch der innere Bereich der teilchenförmigen Polymergebilde mit dem Lösungsmittel oder - gemisch und somit dem Nachvernetzer in Kontakt gebracht werden.

Als Nachvernetzer, die im erfindungsgemäßen Verfahren eingesetzt werden, werden vorzugsweise Verbindungen verstanden, die mindestens zwei funktionelle Gruppen aufweisen, die mit funktionellen Gruppen eines Polymergebildes in einer Kondensationsreaktion (=Kondensationsvemetzer), in einer Additionsreaktion oder in einer Ringöffnungsreaktion reagieren können. Als Nachvernetzer sind im erfindungsgemäßen Verfahren diejenigen bevorzugt, die in WO 2004/037903 A2 als Vernetzer der Vernetzerklassen II genannt wurden.

Unter diesen Verbindungen sind als Nachvernetzer besonders bevorzugt Kondensationsvemetzer wie beispielsweise Diethylenglykol, Triethylenglykol, Polyethylenglykol, Glyzerin, Polyglyzerin, Propylenglykol, Diethanolamin, Triethanolamin, Polyoxypropylen, Oxyethylen-Oxypropylen-Blockcopolymere, Sorbitanfettsäureester, Polyoxyethylensorbitanfettsäureester, Trimethylolpropan, Pentaerythrit, Polyvinylalkohol, Sorbitol, 1,3-Dioxolan-2-on (Ethylencarbonat), 4-Methyl-1,3-dioxolan-2-on (Propylencarbonat), 4,5-Dimethyl-1,3-dioxolan-2-on, 4,4-Dimethyl-1,3-dioxolan-2-on, 4-Ethyl-1,3-dioxolan-2-on, 4-Hydroxymethyl-1, 3-dioxolan-2-on, 1,3-Dioxan-2-on, 4-Methyl-1,3-dioxan-2-on, 4,6-Dimethyl-1,3-dioxan-2-on sowie besonders bevorzugt 1,3-Dioxolan-2-on.

Nachdem die Polymergebilde bzw. die Hydrogele mit dem Nachvernetzer bzw. mit dem Fluid beinhaltend den Nachvernetzer in Kontakt gebracht wurden, werden sie auf eine Temperatur in einem Bereich von 50 bis 300°C, vorzugsweise 75 bis 275°C und besonders bevorzugt 150 bis 250°C erhitzt, so dass vorzugsweise der Aussenbereich der Polymergebilde im Vergleich zum Innenbereich stärker versetzt wird (=Nachvernetzung). Die Zeitdauer der Wärmebehandlung wird durch die Gefahr, dass das gewünschte Eigenschaftsprofil der Polymergebilde infolge von Hitzeeinwirkung zerstört wird, begrenzt.

In einer Ausgestaltung der erfindungsgemäßen Zusammensetzung weist das Polymergebilde
- einen Innenbereich, sowie
- einen den Innenbereich umgebenden Außenbereich
auf,
wobei der Außenbereich einen höheren Vernetzungsgrad aufweist als der Innenbereich. In diesem Zusammenhang ist es bevorzugt, dass die Konzentration des Geruchsbinders in oder auf dem Außenbereich höher ist als die Konzentration des Geruchsbinders in dem Innenbereich. Somit bildet sich sowohl in Hinblick auf die Vernetzung als auch auf die Geruchsbinderkonzentration eine Art Kem-Schale-Morphologie in der erfindungsgemäßen Zusammensetzung heraus. Dieses gilt insbesondere, wenn sich der Geruchsbinder auf der Oberfläche des wasserabsorbierenden Polymergebildes ggf. mit nur geringer Eindringtiefe (meist weniger als 2% des Durchmessers des wasserabsorbierenden Polymergebildes) in dem wasserabsorbierenden Polymergebilde befindet. Dieses kann insbesondere dadurch erreicht werden, in dem der Geruchsbinder am Ende oder nach der Oberflächenvernetzung zugegeben wird.

In einer anderen Ausgestaltung der erfindungsgemäßen Zusammensetzung weist das Polymergebilde
- einen Innenbereich, sowie
- einen den Innenbereich umgebenden Außenbereich
auf,
wobei die Konzentration des Geruchsbinders in oder auf dem Außenbereich höher ist als die Konzentration des Geruchsbinders in dem Innenbereich.

In einer weiteren Ausführungsform des erfindungsgemäßen Verfahrens erfolgt das Konfektionieren des wasserabsorbierenden Polymergebildes oder der superabsorbierenden Zusammensetzung durch in Kontakt bringen mit Metallsalzen und/oder mit einer Kombination aus dem Metallsalz und dem Oxid eines Metalls und/oder Modifizierungsmittel bevor das wasserabsorbierende Polymergebilde mit einem in WO 2004/037903 A2 als Vernetzer der Vernetzerklassen II genannten Nachvernetzer in Kontakt gebracht worden ist.

Zudem betrifft die Erfindung ein Verfahren zur Herstellung einer superabsorbierenden Zusammensetzung, beinhaltend die Verfahrensschritte:
I. Bereitstellen eines wasserabsorbierenden Polymergebildes, das vorzugsweise in der vorstehend beschriebenen Weise erhalten werden kann;
II. Inkontaktbringen der Oberfläche des wasserabsorbierenden Polymergebildes mit einem Geruchsbinder beinhaltend eine Komponente B1 oder eine Komponente B2.

Im Zusammenhang mit den Komponenten B1 und B2 wird auf die vorstehenden Ausführungen zu den Komponenten b1 und b2 Bezug genommen, wobei das in diesem Zusammenhang ausgeführte ebenso für die Komponenten B1 und B2 gilt.

Das Inkontaktbringen kann allgemein durch jede dem Fachmann geeignet erscheinende Methode erfolgen. Nach einer Ausgestaltung des erfindungsgemäßen Verfahrens erfolgt das in Kontakt bringen durch das Mischen von wasserabsorbierenden Polymergebilden mit dem Geruchsbinder. In diesem Zusammenhang ist es bevorzugt, dass sowohl das wasserabsorbierende Polymergebilde als auch der oder die Geruchsbinder in Pulverform und damit als Teilchen vorliegen. Es hat sich weiterhin bewährt, dass bei dem Zusammenmischen von dem als Pulver vorliegenden wasserabsorbierenden Polymergebilde und dem ebenso als Pulver vorliegenden Geruchsbinder ERT 420.2-02 mittleren Teilchengrößen sich unterscheiden. Dabei sollte das wasserabsorbierende Polymergebilde eine größere Teilchengröße als der Geruchsbinder aufweisen. Vorzugsweise unterscheiden sich die Teilchengrößen, vorzugsweise die mittleren Teilchengrößen gemäß ERT 420.2-02, des wasserabsorbierenden Polymergebildes und des Geruchsbinders um mindestens einen Faktor 2 und weiterhin bevorzugt um mindestens einen Faktor 5 sowie darüber hinaus bevorzugt um mindestens einen Faktor 10.

In einer Ausgestaltung des erfindungsgemäßen Verfahrens kann das wasserabsorbierende Polymergebilde auf weniger als 40 Gew.%, vorzugsweise weniger als 30 Gew.-%, besonders bevorzugt weniger als 20 Gew.-% und darüber hinaus bevorzugt weniger als 10 Gew.%, jeweils bezogen auf das wasserabsorbierende Polymergebilde, Superabsorberfeinteilchen mit einer Partikelgröße von weniger als 150 µm, bestimmt nach ERT 420.1-99, basieren.

In einer anderen Ausführungsform des erfindungsgemäßen Verfahrens kann das wasserabsorbierende Polymergebilde mindestens 60 Gew.-%, vorzugsweise mindestens 70 Gew.-%, besonders bevorzugt mindestens 80 Gew.-% und darüber hinaus bevorzugt mindestens 90 Gew.%, jeweils bezogen auf das wasserabsorbierende Polymergebilde, Teilchen mit einer Teilchengröße in einem Bereich von 150 bis 850 µm, bestimmt nach ERT 420.1-99, aufweisen.

In einer anderen Ausführungsform des erfindungsgemäßen Verfahrens kann weniger als 20 Gew.% vorzugsweise weniger als 10 Gew.% und besonders bevorzugt weniger als 5 Gew.-%, jeweils bezogen auf die Zusammensetzung, eines vernetzbare, nicht vernetzten Polymers als ein Binder eingesetzt werden.

In einer anderen Ausgestaltung des erfindungsgemäßen Verfahrens ist es bevorzugt, dass der Geruchsbinder zusammen mit einer Flüssigkeit, vorzugsweise Wasser, Isopropanol, Ethanol oder Aceton und darüber hinaus bevorzugt Wasser, auf das wasserabsorbierende Polymergebilde aufgebracht wird. Die hierbei verwendete Flüssigkeitsmenge liegt vorzugsweise in einem Bereich von 0,1 bis 20 Gew.-%, darüber hinaus bevorzugt in einem Bereich von 1 bis 15 Gew.-% und ferner bevorzugt in einem Bereich von 3 bis 17 Gew.-%, jeweils bezogen auf das Gewicht des trockenen wasserabsorbierenden Polymergebildes. Es ist weiterhin in manchen Fällen hilfreich, ein Lösungsmittel oder -gemisch bei dem Vermischen des Geruchsbinders mit dem wasserabsorbierenden Polymergebilde zur verwenden. In diesem Zusammenhang sind die vorstehend beschriebenen Lösungsmittel oder - gemische und die dort beschriebenen Konzentrationen ebenso bevorzugt. Es sollte dabei darauf geachtet werden, so wenig wie möglich Lösungsmittel oder -gemisch zu verwenden, da dieses ggf. durch Trocknen wieder entfernt werden muss.

Das Inkontaktbringen des Geruchsbinders kann zum einen schon bei der Herstellung des wasserabsorbierenden Polymergebildes erfolgen, in dem der Geruchsbinder, insbesondere der Geruchsbinder B2, vor allen Dingen dann wenn dieser eine Doppelbindung aufweist, bereits den zur radikalischen Polymerisation des wasserabsorbierenden Polymergebildes verwendeten Edukten beigemischt wird. Hierdurch kann erreicht werden, dass der Geruchsbinder und insbesondere die Komponente B2, vor allen Dingen, wenn sie diese Doppelbindungen aufweist, möglichst homogen und mit dem wasserabsorbierenden Polymergebilde fest verbunden eingearbeitet ist.

In einer anderen Ausgestaltung des erfindungsgemäßen Verfahrens erfolgt das Inkontaktbringen des Geruchsbinders mit dem wasserabsorbierenden Polymergebilde, wobei das wasserabsorbierende Polymergebilde als Hydrogel vorliegt. Unter Hydrogel wird allgemein ein mit Wasser oder einer wässrigen Lösung gequollenes wasserabsorbierendes Polymergebilde verstanden. Zur Bearbeitung im Zusammenhang mit dem erfindungsgemäßen Verfahren bevorzugte Hydrogele weisen einen Gehalt an Wasser bzw. wässrigen Flüssigkeiten von mindestens 10 Gew.-%, vorzugsweise in einem Bereich von 20 bis 200 Gew.-% der Flüssigkeit, jeweils bezogen auf das Trockengewicht des wasserabsorbierenden Polymergebildes auf. Auch im Zusammenhang mit dem Aufbringen des Geruchsbinders auf ein als Hydrogel vorliegendes wasserabsorbierendes Polymergebilde ist es bevorzugt, dass das Hydrogel als Granulat vorliegt, so dass die im Vergleich zur Größe dieser Granulatteilchen wesentlich kleineren Geruchsbinderteilchen, die ebenso als Aufschwemmung oder gar gelöst in einer Flüssigkeit, vorliegen können, möglichst gleichmäßig auf der Oberfläche der Hydrogelgranulate verteilt werden können. Bei einer weiteren Ausgestaltung des erfindungsgemäßen Verfahrens, in dem das wasserabsorbierende Polymergebilde bei dem Inkontaktbringen mit dem Geruchsbinder als Hydrogel vorliegt, ist es bevorzugt, auch andere zur Nachbehandlung des Hydrogels verwendete Substanzen einzusetzen, wobei die oben beschriebenen Nachvernetzer besonders bevorzugt sind. In dem Fall, dass Nachvernetzer zusätzlich zu den Geruchsbindern auf das Hydrogel aufgebracht werden, wird die - gleichfalls zuvor beschriebene - thermische Behandlung zur Nachvernetzung nach dem Inkontaktbringen durchgeführt.

Im Fall des Inkontaktbringens des wasserabsorbierenden Polymergebildes mit einer Aminosäure, insbesondere mit Cystein ist es bevorzugt, dass dieses bei Temperaturen von unter 150°C vorzugsweise unter 100°C und besonders bevorzugt unter 80°C erfolgt. Eine besonders die Geruchsbindungseigenschaften des eine Aminosäure, insbesondere Cystein, beinhaltenden Geruchsbinders schonende Vorgehensweise sieht eine beim Inkontaktbringen und den im Fall der Verwendung einer meist wässrigen Aminosäure beinhaltenden Lösung dann folgenden Trockenschritt und ggf. weiterhin folgenden Bearbeitungsschritten vor, dass mindestens das in Kontakt bringen, vorzugsweise mindestens das Inkontaktbringen und das Trocknen, bei Temperaturen in einem Bereich von 15 bis < 80°C und vorzugsweise in einem Bereich von 20 bis 75°C sowie darüber hinaus bevorzugt in einem Bereich von 25 bis 70°C erfolgen. Es ist weiterhin möglich zum Unterstützen des Trocknens einen Unterdruck anzulegen, der üblicherweise in einem Bereich von Normaldruck bis 10 Torr liegt.

Einer anderen Ausgestaltung des erfindungsgemäßen Verfahrens folgend wird zu dem Inkontaktbringen mit dem Geruchsbinder ein wasserabsorbierendes Polymergebilde mit einem Wassergehalt von weniger als 10 Gew.-%, vorzugsweise weniger als 7 Gew.-% und darüber hinaus bevorzugt weniger als 6 Gew.-%, jeweils bezogen auf das trockene Polymergebilde, eingesetzt. Der Wassergehalt kann nach der EDANA-Methode ERT 430.1-99 bestimmt werden. Bei dem wasserabsorbierenden Polymergebilde mit dem geringen Wassergehalt kann es sich nach einer Ausgestaltung des erfindungsgemäßen Verfahrens um ein noch nicht nachvernetztes wasserabsorbierendes Polymergebilde und nach einer anderen Ausgestaltung des erfindungsgemäßen Verfahrens um ein bereits nachvernetztes wasserabsorbierendes Polymergebilde handeln.

Für den Fall, dass der Geruchsbinder mit dem wasserabsorbierenden Polymergebilde mit Hilfe einer Flüssigkeit in Kontakt gebracht wird, ist es in dem erfindungsgemäßen Verfahren bevorzugt, die so erhaltene superabsorbierende Zusammensetzung zumindest teilweise von der eingesetzten Flüssigkeit durch Trocknen zu befreien. Bei der Auswahl der Bedingung für diesen Trockenvorgang sollte darauf geachtet werden, dass der Geruchsbinder in seiner geruchsbindenden Aktivität nicht eingeschränkt wird. Üblicherweise liegen die Trockentemperaturen in einem Bereich von 30 bis 200°C, vorzugsweise in einem Bereich von 50 bis 150°C und darüber hinaus bevorzugt in einem Bereich von 70 bis 120°C. Der Trockenvorgang kann über mindestens 5 Minuten, vorzugsweise mindestens 10 Minuten und darüber hinaus bevorzugt in einem Bereich von 15 bis 180 Minuten erfolgen. Auf eine geeignete Auswahl der Trocknungsbedingungen ist gleichfalls bei dem Fall zu achten, bei dem der Geruchsbinder zusammen mit einem Nachvernetzer mit dem wasserabsorbierenden Polymergebilde in Kontakt gebracht wird und die Nachvernetzung nach dem Inkontaktbringen erfolgen soll.

Weiterhin entspricht es einer Ausgestaltung des erfindungsgemäßen Verfahrens, dass das Inkontaktbringen weiterhin III in Gegenwart eines polymeren Binders erfolgt. Im Zusammenhang mit dem polymeren Binder III wird auf die Ausführungen zu dem polymeren Binder iii verwiesen, wobei die vorstehend gemachten Ausführungen ebenfalls für den polymeren Binder III gelten. Der polymere Binder wird vorzugsweise dann in dem erfindungsgemäßen Verfahren eingesetzt, wenn der Geruchsbinder nicht als Flüssigkeit, sondern als Feststoff aufgetragen wird. Die Auftragung des polymeren Binders kann sowohl auf das als Hydrogel vorliegende wasserabsorbierende Polymergebilde als auch auf das getrocknete wasserabsorbierende Polymergebilde und insbesondere auf das bereits nachvernetzte wasserabsorbierende Polymergebilde jeweils zusammen mit dem Geruchsbinder erfolgen. Es entspricht einer bevorzugten Ausgestaltung des erfindungsgemäßen Verfahrens, dass das Inkontaktbringen des Geruchsbinders oder des Geruchsbinders mit dem polymeren Binder mit einem wasserabsorbierenden Polymergebilde erfolgt, wobei das wasserabsorbierende Polymergebilde vorzugsweise nicht als Hydrogel, sondern wie vorgehend beschrieben trocken vorliegt. Anders ausgedrückt ist es bevorzugt, dass das wasserabsorbierende Polymergebilde vor dem Inkontaktbringen oberflächenvemetzt wird. Hierbei ist es bevorzugt, dass diese Oberflächenvernetzung nicht nur das Aufbringen des Oberflächenvernetzers sondern auch die Oberflächenvernetzungsreaktion, die in der Regel durch thermisches Behandeln erfolgt, einschließt.

Einen weiteren Beitrag zur Lösung der eingangs beschriebenen Aufgaben liefern die durch das erfindungsgemäße Verfahren erhältlichen superabsorbierenden Zusammensetzungen.

Die erfindungsgemäße superabsorbierende Zusammensetzung weist bevorzugt als Eigenschaft
- eine Retention nach ERT 441-2-02 von mindestens 20g/g, vorzugsweise mindestens 25g/g und besonders bevorzugt in einem Bereich von 25 bis 50 g/g;
- eine Absorption unter Druck von 0,7 psi von mindestens 10g/g, vorzugsweise 13g/g und besonders bevorzugt in einem Bereich von 13 bis 30g/g auf.

Dabei ist es bevorzugt, dass die durch das erfindungsgemäße Verfahren erhältlichen erfindungsgemäßen superabsorbierenden Zusammensetzungen die gleichen Eigenschaften aufweisen, wie vorstehend für die superabsorbierenden Zusammensetzungen beschrieben. Es ist auch erfindungsgemäß bevorzugt, dass diejenigen Werte, die im Zusammenhang mit dem erfindungsgemäßen Verfahren und den erfindungsgemäßen Polymergebilden sowie den superabsorbierenden Zusammensetzungen als Untergrenzen von erfindungsgemäßen Merkmalen ohne Obergrenzen angegeben wurden, das 20-fache, vorzugsweise das 10-fache und besonders bevorzugt das 5-fache des am meisten bevorzugten Wertes der Untergrenze besitzen.

Einen weiteren Beitrag zur Lösung der eingangs beschriebenen Aufgaben liefert ein Verbund, beinhaltend die erfindungsgemäßen wasserabsorbierenden Polymergebilde bzw. die durch das erfindungsgemäße Verfahren erhältlichen oberflächenbehandelten wasserabsorbierenden Polymergebilde und ein Substrat. Es ist dabei bevorzugt, dass die erfindungsgemäßen Polymergebilde und das Substrat miteinander fest verbunden sind. Als Substrate sind Folien aus Polymeren, wie beispielsweise aus Polyethylen, Polypropylen oder Polyamid, Metalle, Vliese, Fluff, Tissues, Gewebe, natürliche oder synthetische Fasern, oder andere Schäume bevorzugt. Weiterhin ist es erfindungsgemäß bevorzugt, dass der Verbund mindestens einen Bereich beinhaltet, welcher das erfindungsgemäße wasserabsorbierende Polymergebilde in einer Menge in einem Bereich von etwa 15 bis 100 Gew.%, vorzugsweise etwa 30 bis 100 Gew.-%, besonders bevorzugt von etwa 50 bis 99,99 Gew.-%, ferner bevorzugt von etwa 60 bis 99,99 Gew.-% und darüber hinaus bevorzugt von etwa 70 bis 99 Gew.%, jeweils bezogen auf das Gesamtgewicht des betreffenden Bereichs des Verbundes, beinhaltet, wobei dieser Bereich vorzugsweise eine Größe von mindestens 0,01 cm³, vorzugsweise mindestens 0,1 cm³ und am meisten bevorzugt mindestens 0,5 cm³ aufweist.

In einer Ausgestaltung des erfindungsgemäßen Verbundes handelt es sich um einen flächenförmigen Verbund, wie er in der WO 02/056812 A1 als "absorbent material" beschrieben ist. Der Offenbarungsgehalt der WO 02/056812 A1, insbesondere hinsichtlich des genauen Aufbaus des Verbundes, des Flächengewichtes seiner Bestandteile sowie seiner Dicke wird hiermit als Referenz eingeführt und stellt einen Teil der Offenbarung der vorliegenden Erfindung dar.

Einen weiteren Beitrag zur Lösung der eingangs genannten Aufgaben liefert ein Verfahren zur Herstellung eines Verbundes, wobei die erfindungsgemäßen superabsorbierende Zusammensetzung bzw. die durch das erfindungsgemäße Verfahren erhältlichen oberflächenbehandelten wasserabsorbierenden Polymergebilde und ein Substrat und gegebenenfalls ein Zusatzstoff miteinander in Kontakt gebracht werden. Als Substrate werden vorzugsweise diejenigen Substrate eingesetzt, die bereits vorstehend im Zusammenhang mit dem erfindungsgemäßen Verbund genannt wurden.

Einen Beitrag zur Lösung der eingangs genannten Aufgaben liefert auch ein Verbund erhältlich nach dem vorstehend beschriebenen Verfahren, wobei dieser Verbund vorzugsweise die gleichen Eigenschaften aufweist wie der vorstehend beschriebene, erfindungsgemäße Verbund.

Nach einem anderen Aspekt der vorliegenden Erfindung ist der Verbund als ein Hygieneartikelcore ausgebildet, das, jeweils bezogen auf das Hygieneartikelcore mindestens 30 Gew.- %, vorzugsweise mindestens 50 Gew.-% und darüber hinaus bevorzugt mindestens 70 Gew.- % der erfindungsgemäßen wasserabsorbierenden Zusammensetzung und mindestens 1 Gew.%, vorzugsweise mindestens 5 Gew.-% und darüber hinaus bevorzugt mindestens 10 Gew.- % des Substrats und gegebenenfalls noch weitere übliche Hilfs- und/oder Haftmittel beinhaltet, wobei die Summe der Gewichtsprozente der einzelnen im Hygieneartikelcore beinhalteten Komponenten an 100 Gew.-% ergibt. Als Substrat im Zusammenhang mit dem Hygieneartikelcore sind besonders Materialien bevorzugt, die zur Fixierung der meist als Teilchen vorliegenden erfindungsgemäßen superabsorbierenden Zusammensetzung dienen. Hierbei kann es sich um Fasern oder Gewirke oder Gewebe sowie Netze handeln. Es ist weiterhin möglich, dass die beispielsweise als Pulver und damit teilchenförmig vorliegende superabsorbierende Zusammensetzung durch ein Haftmittel wie einen Klebstoff mit dem Substrat verbunden ist. Gleichfalls entspricht es einer Ausgestaltung, dass das Substrat so ausgestaltet ist, dass die superabsorbierende Zusammensetzung in Ausnehmung des Substrats aufgenommen wird. Übliche, ebenfalls in dem Hygieneartikelcore einarbeitbare Hilfsmittel sind beispielsweise Stoffe, kosmetische Stoffe, die die Hautverträglichkeit erhöhen, Desinfektionsmittel, antimikrobielle Substanzen und dergleichen.

In einem weiteren Aspekt betrifft die vorliegende Erfindung Hygieneartikel, beinhaltend eine flüssigkeitsdurchlässige Oberschicht, eine flüssigkeitsunzulässige Unterschicht und einen zwischen der Oberschicht und der Unterschicht angeordneten erfindungsgemäßen Verbund. Als Hygieneartikel kommen sowohl Damenhygieneartikel, erwachsene Inkontinenzprodukte als auch Windeln für Säuglinge, Babys und Kleinkinder in Betracht. Bevorzugt ist es, dass der Hygieneartikel ein vorstehend beschriebenes Hygieneartikelcore beinhaltet. Als flüssigkeitsdurchlässige Oberschicht kommen grundsätzlich alle dem Fachmann hierfür bekannten und geeignet erscheinenden Gewebe, Gelege und Gewirklagen, die meist aus Zellstoffen oder Zellstoffderivaten bestehen, die gegebenenfalls mit Kunststoffen wie Polypropylen oder Polyethylen boniert sind, in Betracht. Auch als flüssigkeitsundurchlässige Unterschicht werden die in der Industrie dem Fachmann geläufigen meist ebenfalls aus einem Zellstoff oder Zellstoffderivat, -gelege, -gewirk, oder -gestrick bestehenden Vliese eingesetzt, wobei diese im Allgemeinen mit einer Kunststoffschicht, meist aus Polypropylen oder Polyethylen, abgelichtet sind.

Einen weiteren Beitrag zur Lösung der eingangs genannten Aufgaben liefern chemische Produkte beinhaltend die erfindungsgemäßen superabsorbierenden Zusammensetzungen oder einen erfindungsgemäßen Verbund. Bevorzugte chemische Produkte sind insbesondere Schäume, Formkörper, Fasern, Folien, Filme, Kabel, Dichtungsmaterialien, flüssigkeitsaufnehmende Hygieneartikel, insbesondere Windeln und Damenbinden, Träger für pflanzen- oder pilzwachstumsregulierende Mittel oder Pflanzenschutzwirkstoffe, Zusätze für Baustoffe, Verpackungsmaterialien oder Bodenzusätze.

Auch die Verwendung der erfindungsgemäßen Polymergebilde oder des erfindungsgemäßen Verbundes in chemischen Produkten, vorzugsweise in den vorstehend genannten chemischen Produkten, insbesondere in Hygieneartikeln wie Windeln oder Damenbinden, sowie die Verwendung der Superabsorberpartikel als Träger für pflanzen- oder pilzwachstumsregulierenden Mittel oder Pflanzenschutzwirkstoffe liefern einen Beitrag zur Lösung der eingangs genannten Aufgaben. Bei der Verwendung als Träger für pflanzen- oder pilzwachstumsregulierende Mittel oder Pflanzenschutzwirkstoffe ist es bevorzugt, dass die pflanzen- oder pilzwachstumsregulierende Mittel oder Pflanzenschutzwirkstoffe über einen durch den Träger kontrollierten Zeitraum abgegeben werden können.

Die Erfindung wird nachfolgend anhand von nichtlimitierenden Beispielen erläutert.

### TESTMETHODEN

### Allgemein

Sofern nicht nachfolgend andere Testmethoden vorgegeben werden, wird auf die dem Fachmann allgemein bekannten und üblich erscheinenden Testmethoden zurückgegriffen, wobei insbesondere Testmethoden der EDANA (European Diaper and Nonwoven Association) Anwendung finden, die allgemein als "ERT-Methoden" angegeben werden.

### Retention und Absorption

Die Retention wurde als CRC (Centrifuge Retention Capacity) nach ERT 441-2-02 an der gesamten Kornfraktion bestimmt, wobei 30 Minuten aufgesogen und direkt danach für 3 Minuten geschleudert wurde. Die Absorption unter Druck wurde als AAP (Absorption Against Pressure) nach ERT 442-2-02 an der gesamten Kornfraktion bestimmt.

### Bestimmung des Wicking-Index

Die Bestimmung des Wicking-Index erfolgte gemäß dem auf der Seite 6, Zeile 36 bis Seite 7, Zeile 26 der EP-A-0 532 002 beschriebenen Testverfahren, wobei die gesamte Kornfraktion und an Stelle des synthetischen Urins eine 0,9%ige wässrige Kochsalzlösung (hergestellt aus *aqua dest*. und NaCl p.a. mit einem pH-Wert in einem Bereich von 5,0 bis 8,0) verwendet wurde.

### Geruchsbindung

Die Geruchsbindung wurde durch Headspace Gaschromatographie (GC) bestimmt. Um die Abnahme von Geruchsstoffen im Dampfraum zu bestimmen, wurde eine Probe superabsorbierender Zusammensetzung je 100,0mg in ein fest verschließbares Druckfläschchen (10ml Headspace-Druckfläschchen Hewlett Packard^{®} Part-No.:9301-0717) eingewogen und mit einer wässrigen Lösung der Geruchssubstanz (1µl einer Stammlösung von 50mmol/l Furfurylmercaptan in Dimethylformamid wurden in 25ml Wasser gegeben) versetzt. Nach einer Wartezeit von 16 h bei Raumtemperatur erfolgte ein nachstehend näher beschriebenes Temperieren der Druckfläschchen im Ofen des Headspace-Probengeber (Hewlett Packard^{®} 7694). Dann wurde aus der Dampfphase des Druckfläschchens in den GC Hewlett Packard^{®} 5890A injiziert. Die Trennung erfolgt auf einer apolaren Kapillarsäule RTX^{®} 5 (Crossbond® 5% diphenyl - 95% Dimethyl-Polysiloxan, Geometrie: 60m * 0.53mm * 1.0µ, Hersteller: Restek Corp.), welche an einen FID-Detektor angeschlossen ist. Im Headspace wurden folgende Bedingungen eingehalten: Ofen-Temp.: 45°C, Loop-Temp.: 120°C, Tr.Line-Temp.: 120°C, GC Cykluszeit: 12,0min, Vial Eq.Zeit: 60,0min, Pressuriz.Zeit: 0,13min, Loop-Einfüllzeit: 0,02min, Loop Eq.Zeit: 0,02min, Injektionszeit: 0,50min, "Shake": Off, Kartuschendruck: 134kPa, Vial-Druck: 152kPa; Temperaturprogramm: 170°C für 0min, -30°C/min auf 110°C für 2min, 35°C/min auf 250°C für 2min; Injektor: Säulen-Fluß: 13ml/sec; Split (ohne HS): 0ml/sec, Split (mit HS): 150ml/sec, Säulenvordruck: 100kPa, Temperatur: 250°C, "Purge": On; Detektor: Detektortemperatur: 250°C, Detektor-Range: 0.

Zur Quantifizierung der Abnahme wird die Peakfäche der Geruchssubstanz der Probe mit der einer Kontrollprobe verglichen, die keinen Geruchsbinder enthält.

### Akquisitionszeit und Flüssigkeitsverteilung

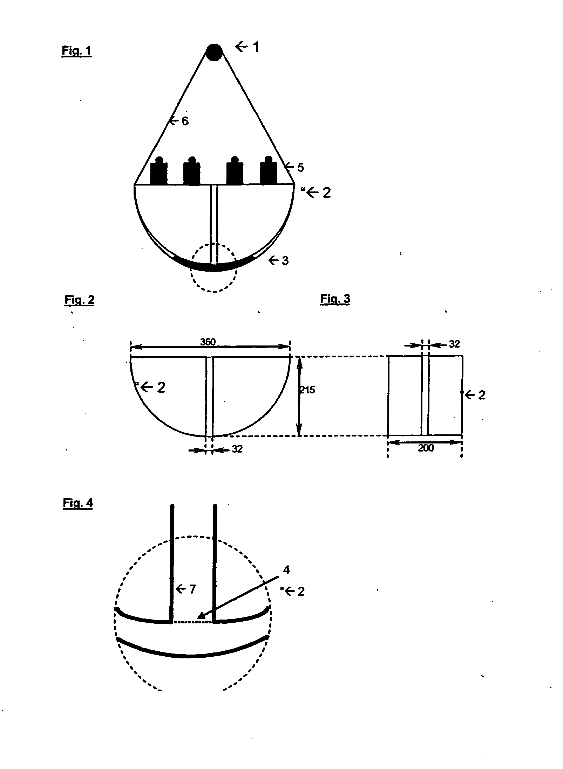

Die Messeinrichtung zur Bestimmung von Akquisitionszeit und Flüssigkeitsverteilung des Cores ist in den Figuren 1 bis 4 schematisch dargestellt. Fig. 1 zeigt einen seitlichen Schnitt durch die Messeinrichtung, wobei an einer als Laborstab ausgebildeten Aufhängung 1 über eine 200mm breite ca. 1mm starke, nicht elastische und glatte Kunststofffolie 6 ein aus transparentem Plexiglas gebildeter einem Gesäß eines Hygieneartikelträgers im Prinzip nachempfundener Einsatz 2 gehalten wird. Der Einsatz 2 ist im wesentlichen ein Halbrund mit einer glatten Oberfläche, das in seinem Zentrum einen Kanal 7 aufweist, der, wie in Figur 4 als Ausschnitt dargestellt über ein Sieb 4 an seiner unteren Öffnung verfügt. Das Core 3 wird mit seinem sich aus der Aufsicht ergebenden Mittelpunkt unter die untere Öffnung des Kanals 7 gegeben. Anschließend wird der Einsatz 2 mit den für die Messungen vorgesehenen Gewichten 5 beschwert. Durch die obere Öffnung des Kanals 7 wird die Testflüssigkeit in die Messeinrichtung gefüllt. Die Figuren 2 und 3 geben die Maße der Messeinrichtung von Vorderansicht und der Seitenansicht in mm an.

Das Core in einer Größe von 300mm Länge x 120mm Breite wurde gewogen, in die körpergeformte Testapparatur gelegt und mit 9 kg belastet. Danach wurden dreimal 60 g einer 0,9%igen wässrigen Kochsalzlösung, die mit 5 ml/l Säurefuchsin Stammlösung (in 11 einer 0,9%igen Kochsalzlösung wurden 10,0g Säurefuchsin hinzugegeben und der pH-Wert der Lösung durch Zugabe von 0,3%iger wässriger NaOH auf 6,0 eingestellt) eingefärbt war, mittels Messzylinder in einem zeitlichen Abstand von 20 Minuten durch die Öffnung der Testapparatur gegeben. Mittels Stoppuhr kann die Einsickerzeit vom Zugabestart bis zum vollständigen Einsickern der Testflüssigkeit gemessen werden. Nach jeder Flüssigkeitszugabe folgt eine Wartezeit von 20 Minuten. Anschließend werden die Gewichte abgenommen. Die Flüssigkeitsverteilung wurde jeweils nach Ablauf der oben genannten 20 Minuten bestimmt. Dazu wurde die Ausbreitung der Flüssigkeit in der Länge des Cores gemessen.

### BEISPIELE

### Beispiel 1:

### (Herstellung eines nicht nachvernetzten wasserabsorbierenden Polymergebildes als Hydrogel - Probe a)

Eine Monomerenlösung bestehend aus 280 g Acrylsäure, die zu 70 Mol-% mit Natronlauge neutralisiert wurde, 466,8 g Wasser, 1,4 g Polyethylenglykol-300-diacrylat und 1,68 g Allyloxypolyethylenglykolacrylsäureester wird durch Spülen mit Stickstoff vom gelösten Sauerstoff befreit und auf die Starttemperatur von 4°C abgekühlt. Nach Erreichen der Starttemperatur wurde die Initiatorlösung (0,3 g Natriumperoxydisulfat in 10 g H₂O, 0,07 g 30%ge Wasserstoffperoxidlösung in 1 g H₂O und 0,015 g Ascorbinsäure in 2 g H₂O) zugesetzt. Nachdem die Endtemperatur von ca. 100°C erreicht war, wurde das entstandene Gel gewölft, so dass ein Granulat mit etwa 1 bis 3mm großen Teilchen als Probe a erhalten wurde. Der Wassergehalt betrug ca. 50 %.

### Beispiel 2:

### (Herstellung eines nicht nachvernetzten wasserabsorbierenden Polymergebildes als Pulver - Probe b)

Probe a wurde bei 150°C 120 Minuten lang getrocknet. Das getrocknete Polymerisat wurde grob zerstoßen, gemahlen und auf ein Pulver mit einer Partikelgröße von 150 bis 850 µm gesiebt und eine Probe b mit einer mittleren Teilchengröße nach ERT 420.2-02 von 520 µm erhalten. Der nach ERT 430.1-99 bestimmte Wassergehalt betrug 5%.

### Beispiel 3:

### (Herstellung eines nachvernetzten wasserabsorbierenden Polymergebildes als Pulver - Probe c)

Zur Nachvernetzung wurden 100g des oben erhaltenen Pulvers der Probe b unter kräftigen Rühren mit einer Lösung aus 1 g 1,3-Dioxalan-2-on, 3g Wasser vermischt und anschließend für 30 Minuten in einem Ofen, der auf 180°C temperiert war, erhitzt. Die so erhaltene Probe c hatte eine mittlere Teilchengröße nach ERT 420.2-02 von 525 µm. Der in Probe c nach ERT 430.1-99 bestimmte Wassergehalt betrug 4,5%.

### Beispiel 4:

### (Superabsorbierende Zusammensetzungen - Geruchsbinder auf Hydrogel mit Nachvernetzung)

100 Trockenmasseteile der Probe a wurden vorgelegt und mit der in Tabelle 1 angegebenen Menge (bezogen auf die Trockenmasseteile) an Geruchsbinder versetzt. Anschließend wurde die Mischung durch Wölfen homogenisiert und bei 150°C 120 Minuten lang getrocknet. Das getrocknete Polymerisat wurde grob zerstoßen, gemahlen und auf ein Pulver mit einer Partikelgröße von 150 bis 850 µm gesiebt und eine Probe b mit einer mittleren Teilchengröße nach ERT 420.2-02 von 515 µm erhalten. Der nach ERT 430.1-99 bestimmte Wassergehalt betrug 5,5%. Zur Nachvernetzung wurden 100g des oben erhaltenen Pulvers unter kräftigen Rühren mit einer Lösung aus 1 g 1,3-Dioxalan-2-on, 3g Wasser vermischt und anschließend für 30 min in einem Ofen, der auf 180°C temperiert war, erhitzt. Das so erhaltene Produkt wurde gemäß der Angaben in Tabelle 1 analysiert. Die eingesetzten Tegosorb^{®}-Typen sind kommerziell bei der Goldschmidt GmbH erhältlich. Die Kontrollprobe wurde ohne den Zusatz des Geruchsbinders genauso wie vorstehend behandelt.

**Tabelle 1**

| Probe | Geruchsbinder Art & Menge (Trockenmasse) | CRC [g/g] | AAP (0,7 psi) [g/g] | Geruchsbindung* [%] |
|---|---|---|---|---|
| 4-1 | 1% Tegosorb^{®} PY88, 1,5% Cystein | 30,3 | 14,2 | 80 |
| 4-2 Vergleichsbeispiel | 1,5% Cystein** | 28,8 | 18,6 | 88 |
| 4-3 | 1% Tegosorb^{®} A30, 1,5% Cystein | -*** | -*** | 83 |

| | | | | |
|---|---|---|---|---|
| * Furfurylmerkaptan-Verringerung in der Gasphase im Vergleich zur nicht mit Geruchsbinder enthaltenden gleichen Kontrollprobe; ** Cystein wurde immer als 15%ige wässrige Lösung eingesetzt; *** Keine Messung | | | | |

Aus Tabelle 1 ist ersichtlich, dass erfindungsgemäße superabsorbierende Zusammensetzungen erhalten werden, die neben guten Absorptionseigenschaften eine hervorragende Geruchsbindung zeigen. Außerdem sind die nach EP-A-0 532 002 bestimmte Wicking Indices der Probe c und der Probe 4-2 im Rahmen der Messungenauigkeit dieses Tests gleich.

### Beispiel 5:

### (Superabsorbierende Zusammensetzungen - Geruchsbinder auf nachvernetztes superabsorbierendes Polymer)

100 Trockenmasseteile der Probe c wurden vorgelegt und mit der in Tabelle 2 angegebenen Menge (bezogen auf die Trockenmasseteile) an Geruchsbinder versetzt und in einem MIT Mischer Typ LM 1,5/5 der Firma Mischtechnik Industrieanlagen GmbH homogenisiert. Die Ergebnisse der Analyse sind in Tabelle 2 dargestellt.

**Tabelle 2**

| Probe | Geruchsbinder Art & Menge (Trockenmasse) | CRC [g/g] | AAP (0,7 psi) [g/g] | Geruchsbindung* [%] |
|---|---|---|---|---|
| 5-1, Vergleichsbeispiel | 2% Tegosorb^{®} PY88, 1 % PEG 10000** | 30,8 | 21 | 24 |
| 5-2 | 1 % Tegosorb^{®} PY88, 1,5% Cystein | 28,8 | 21,3 | 88 |
| 5-3 Vergleichs beispiel | 1,5% Cystein*** | 29,4 | 22,5 | 74 |
| 5-4 | 1% Tegosorb^{®} A30, 1,5% Cystein | 27,1 | 20,9 | 83 |

| | | | | |
|---|---|---|---|---|
| * Furfurylmerkaptan-Verringerung in der Gasphase im Vergleich zur nicht mit Geruchsbinder enthaltenden Kontrollprobe (Probe c); ** wurde als 25%ige wässrige Lösung eingesetzt; *** Cystein wurde immer als 15%ige wässrige Lösung eingesetzt; | | | | |

Aus Tabelle 2 ist ersichtlich, dass erfindungsgemäße superabsorbierende Zusammensetzungen erhalten werden, die neben guten Absorptionseigenschaften eine hervorragende Geruchsbindung zeigen. Außerdem sind die Wicking Indices der Probe c (20,8cm) und der Probe 5-3 (19,8cm) im Rahmen der Messungenauigkeit dieses Tests gleich.

### Beispiel 6:

### (Superabsorbierende Zusammensetzungen - Geruchsbinder auf nachvernetztes superabsorbierendes Polymer)

100 Trockenmasseteile des durch die Stockhausen GmbH kommerziell erhältlichen, im Wesentlichen auf schwachvernetzter, mit ca. 75 Mol-% teilneutralisierter Acrylsäure basierenden, nachvernetzten superabsorbierenden Polymers FAVOR^{®} SXM 9155, kommerziell erhältlich durch die Stockhausen GmbH, wurden vorgelegt und mit der in den Tabellen 3 angegebenen Menge (bezogen auf die Trockenmasseteile) an Geruchsbinder versetzt und in dem vorstehend bezeichneten MIT Mischer homogenisiert.

Die Cores wurden mittels einer Mischung von 48,6 Gew.-% einer in den Tabellen 3 angegebenen und hier vorstehend dargestellten superabsorbierenden Zusammensetzung bezogen auf das Core, und 48,6 Gew.-% Zellulosefasern Stora Fluff EF semitreated der Firma Stora-Enzo AB Schweden, sowie 2,8 Gew.-% einer Zweikomponentenfaser aus jeweils 50 Gew.-% Polypropylen (PP) bzw. Polyethylen (PE) mit PP Kern und PE Mantel der Firma Fibervision A/S Dänemark durch ein Airlaid-Verfahren mit einer M&J-Maschine (Breite 40cm, Arbeitsbreite 36cm, Betriebseinstellungen: Bandgeschwindigkeit 3 m/min, Fluffeinzug an Hammermühle 3,1 m/min, Polymerdosierung 380 g/min, Zweikomponentenfaser in 10g Portionen ca. 1 mal/min abgeworfen) gebildet, wobei das absorbierende Polymer homogen eingetragen wurde. Cores mit einem Basisgewicht von 756 g/m², eine Dichte von 0,2 g/cm³ wurden für die Prüfungen verwendet. Die Cores hatte ein Länge von 300 mm und eine Breite von 120 mm. Die Ergebnisse der Core-Analyse sind in Tabellen 3a und 3b dargestellt.

**Tabelle 3a**

| Akquisitionszeit als 6-Fachbestimmung | | | | |
|---|---|---|---|---|
| Probe | Geruchsbinder Art & Menge (Trockenmasse) | 1. Insult | 2. Insult | 3. Insult |
| 6-1 | 2% Cystein* Vergleichsbeispiel | 62s / 10,3g/10s | 493s / 1,3g/10s | 922s / 0,7g/10s |
| 6-2 | 1 % Tegosorb^{®} A30, 2% Cystein | 47s / 13,1g/10s | 324s / 1,9g/10s | 672s / 0,9g/10s |
| 6-3 | 2% Tegosorb^{®} A30 Vergleichsbeispiel | 55s / 11,1g/10s | 377s / 1,6g/10s | 775s / 0,8g/10s |
| 6-4 | SXM 9155 - Kontrolle | 79s / 10,3g/10s | 758s / 0,8g/10s | 1352s / 0,5g/10s |

| | | | | |
|---|---|---|---|---|
| * Cystein wurde immer als 15%ige wässrige Lösung eingesetzt; | | | | |

Aus Tabelle 3a ist ersichtlich, dass erfindungsgemäße superabsorbierende Zusammensetzungen erhalten werden, die neben einer hervorragenden Geruchsbindung auch gegenüber der keinen Geruchsbinder aufweisenden Kontrolle deutlich verbesserte Akquisitionszeiten zeigen.

**Tabelle 3b**

| Flüssigverteilung als 6-Fachbestimmung | | | | |
|---|---|---|---|---|
| Probe | Geruchsbinder Art & Menge (Trockenmasse) | 1. Insult | 2. Insult | 3. Insult |
| 6-1 | 2% Cystein* Vergleichsbeispiel | 11,0cm / 37% | 14,2cm / 47% | 19,5cm / 65% |
| 6-2 | 1% Tegosorb^{®} A30, 2% Cystein | 12,0cm / 40% | 15,8cm / 53% | 21,0cm / 70% |
| 6-3 | 2% Tegosorb^{®} A30 Vergleichsbeispiels | 12,2cm / 41% | 16,3cm / 54% | 20,7cm / 69% |
| 6-4 | SXM 9155 - Kontrolle | 11,2cm / 37% | 13,3cm / 44% | 18,5cm / 62% |

| | | | | |
|---|---|---|---|---|
| * Cystein wurde immer als 15%ige wässrige Lösung eingesetzt; | | | | |

Aus Tabelle 3b ist ersichtlich, dass erfindungsgemäße superabsorbierende Zusammensetzungen erhalten werden, die neben einer hervorragende Geruchsbindung auch gegenüber der keinen Geruchsbinder aufweisenden Kontrolle deutlich verbesserte Flüssigkeitsverteilung zeigen.

### Beispiel 7:

### (Nachvernetztes wasserabsorbierendes Polymergebilde mit Geruchsbinder - Probe e)

100 Trockenmasseteile einer Probe d (hergestellt wie Probe c nur dass der Neutralisationsgrad 50 Mol-% betrug) wurden mit 1 Trockenmasse-% Tegosorb^{®} A 30 als (a) 15%ige oder (b) 25%ige Lösung vermischt, in zylindrischen Glasflaschen für 30 Minuten auf einer Rollbank bei einer Rollgeschwindigkeit von 80 U/min bei Raumtemperatur gemischt und bei Raumtemperatur über 24 h getrocknet. Es wurde im Fall (a) ein Pulver mit einer Partikelgröße von 150 bis 850 µm gesiebt und eine Probe e-a mit einer mittleren Teilchengröße nach ERT 420.2-02 von 517 µm erhalten. Der nach ERT 430.1-99 bestimmte Wassergehalt betrug 7%. Es wurde im Fall (b) ein Pulver mit einer Partikelgröße von 150 bis 850 µm gesiebt und eine Probe e-b mit einer mittleren Teilchengröße nach ERT 420.2-02 von 521 µm erhalten. Der nach ERT 430.1-99 bestimmte Wassergehalt betrug 6,9%.

### Beispiel 8 Vergleichs beispiel

### (Nachvernetztes wasserabsorbierendes Polymergebilde mit Cystein als Geruchsbinder - Probe f)

100 Trockenmasseteile des durch die Stockhausen GmbH kommerziell erhältlichen, im Wesentlichen auf schwachvemetzter, mit ca. 75 Mol-% teilneutralisierter Acrylsäure basierenden, nachvernetzten superabsorbierenden Polymers FAVOR^{®} SXM 9155, kommerziell erhältlich durch die Stockhausen GmbH, wurden vorgelegt und mit 2 Trockenmasseteilen Cystein als 15%ige wässrige Lösung als Geruchsbinder versetzt und in dem vorstehend bezeichneten MIT homogenisiert. Anschließend wurde die Mischung bei 30°C im Wasserstrahlpumpenvakuum getrocknet, bis der nach ERT 430.1-99 bestimmte Wassergehalt 6,5% betrug. Es wurde ein Pulver mit einer Partikelgröße von 150 bis 850 µm gesiebt und eine Probe f mit einer mittleren Teilchengröße nach ERT 420.2-02 von 523 µm erhalten.

### Beispiel 9:

### (Herstellung eines nachvernetzten agglomerierten wasserabsorbierenden Polymergebildes - Probe g)

Die Herstellung von Probe b wurde bis auf den Mahl- und Absiebschritt in einem Maßstab von 5 kg wiederholt. Das so erhaltene Produkt wurde in einer Zentrifugalmühle der Firma Retsch vom Typ ZM100 gemahlen und auf eine erste Fraktion F1 mit einer Partikelgröße von kleiner 150 µm und eine zweite Fraktion F2 mit einer Partikelgröße von 150 bis 850 µm mit einer mittleren Teilchengröße nach ERT 420.2-02 von 521 µm mit einer Analyseabsiebmaschine der Firma Retsch vom Typ AS200 control 'g' abgesiebt.

Zur Herstellung des Binders wurde in einem mit Stickstoff gespülten Glasreaktor als Vorlage 208,16 g 50 %ige NaOH, 842,20 g entionisiertes Wasser, 375,00 g Acrylsäure mit einem Initiatorsystem aus 1,12 g Mercatoethanol, 0,22 g Ascorbinsäure in 9,78 g entionisiertem Wasser und 2,92 Wasserstoffperoxid 35 %ig mit einem Nachkatalysator aus 13,90 g Wasserstoffperoxid 35 %ig, 2,42g Hydroxy-ammoniumchlorid in 7,58 g entionisiertem Wasser sowie 36,70 g des Vernetzers PEG 300 zur Reaktion gebracht. Dazu wurde die Vorlage 1 h mit Stickstoff gespült und bei 25°C in vorstehender Reihenfolge die Bestandteile des Initiator zugegeben, so dass nach 5 Minuten eine Reaktionstemperatur von 90°C erreicht und über 30 Minuten gehalten wurde, bevor die Nachkatalyse zugegeben und nochmals für 1 h gerührt wurde, wonach der Ansatz auf 50°C abgekühlt wurde.

Zur Agglomeration wurde eine Mischung von 200 g bestehend aus 40 Gew.-% der Fraktion F1 und 60 Gew.% der Fraktion F2 in einem MIT-Mischer Typ LM 1,5/5 der Firma Mischtechnik Industrieanlagen GmbH vorgelegt und mit 25 Gew.% (bezogen auf die Mischung) einer 25 %igen wässrigen Lösung des vorstehend hergestellten Binders über eine Spritzenkanüle über 2 Minuten zugegeben und über 1 Minuten zu einem innigen Gemisch vermischt.

Das so erhaltene Gemisch wurde in einem Umlufttrockenschrank bei 180°C für 30 Minuten getrocknet und in einer Zentrifugalmühle der Firma Retsch vom Typ ZM100 gemahlen und auf eine Fraktion mit einer Partikelgröße von 150 bis 850 µm mit einer mittleren Teilchengröße nach ERT 420.2-02 von 522 µm mit einer Analyseabsiebmaschine der Firma Retsch vom Typ AS200 control 'g' als Agglomerat abgesiebt.

Zur Nachvernetzung wurden 100g des oben erhaltenen Agglomerats unter kräftigem Rühren mit einer Lösung aus 1 g 1,3-Dioxalan-2-on und 3g Wasser vermischt und anschließend für 30 Minuten in einem Ofen, der auf 180°C temperiert war, erhitzt. Die so erhaltene Probe g hatte eine mittlere Teilchengröße nach ERT 420.2-02 von 524 µm. Der in Probe g nach ERT 430.1-99 bestimmte Wassergehalt betrug 4,4%.

### Beispiel 10 Vergleichs beispiel

### (Herstellung eines nachvernetzten agglomerierten wasserabsorbierenden Polymergebildes mit Zinkrezinolat - Probe h)

Hierzu wurde Beispiel 9 mit dem Unterschied gefolgt, dass der wässrigen Binderphase vor der Zugabe des Binders zu der Mischung, bezogen auf die Mischung 1 % Zinkrezinolat als Tegosorb^{®} A30 zugemischt wurde. Die so erhaltene Probe h hatte eine mittlere Teilchengröße nach ERT 420.2-02 von 523 µm. Der in Probe h nach ERT 430.1-99 bestimmte Wassergehalt betrug 4,5 %.

### Beispiel 11:

### (Superabsorbierende Zusammensetzungen - Geruchsbinder auf nachvernetztes superabsorbierendes Polymer - Probe il bis i5)

100 Trockenmasseteile der Probe c wurden vorgelegt und mit der in Tabelle 4 angegebenen Menge (bezogen auf die Trockenmasseteile) an Geruchsbinder versetzt und in einem MIT Mischer Typ LM 1,5/5 der Firma Mischtechnik Industrieanlagen GmbH homogenisiert. Die Ergebnisse der Analyse sind in Tabelle 4 dargestellt.

**Tabelle 4**

| (jeweils Dreifachbestimmungen) | | | | |
|---|---|---|---|---|
| Probe | Zn-Rizenolat [%] | AAP 0,7 [g/g] | Oberflächenspannung [mN/m] | Wicking-Index [mm] |
| g Vergleichsbeispiele | 0 | 10,8 | 71,0 | 165 |
| h Vergleichsbeispiele | 1 | 7,7 (-28%) | 39,2 (-45%) | 90 (-45,5%) |
| i 1 Vergleichsbeispiele | 0 | 25,9 | 69,3 | 230 |
| i 2 Vergleichsbeispiele | 0,1 | 24,8 | 41,5 | 165 |
| i 3 Vergleichsbeispiele | 0,3 | 25,2 | 40,9 | 165 |
| i 4 Vergleichsbeispiele | 0,5 | 24,9 | 41,1 | 160 |
| i 5 Vergleichsbeispiele | 1 | 23,9 (-7,7%) | 39,9 (-42,5%) | 170 (-26%) |

## Patentansprüche

1. Geruchsabsorbierende superabsorbierende Zusammensetzung, beinhaltend
i. ein wasserabsorbierendes Polymergebilde, worin das eingesetzte wasserabsorbierende Polymergebilde aus (α1) 20-99,999 Gew.-%, bevorzugt 55-98,99 Gew.-% und besonders bevorzugt 70-98,79 Gew.% polymerisierten, ethylenisch ungesättigten, säuregruppen-tragenden Monomeren oder deren Salze oder polymerisierten, ethylenisch ungesättigten, einen protonierten oder quarternierten Stickstoff beinhaltenden Monomeren, oder deren Mischungen, wobei mindestens ethylenisch ungesättizte, säuregruppenhaltige Monomere, vorzugsweise Acrylsäure, beinhaltende Mischungen besonders bevorzugt sind,
(α2) 0-80 Gew.-%, vorzugsweise 0-44,99 Gew.% und besonders bevorzugt 0,1-44,89 Gew.-% polymerisierten, monoethylenisch ungesättigten, mit (α1) copolymerisierbaren Monomeren,
(α3) 0,001-5 Gew.-%. vorzugsweise 0,01-3 Gew.-% und besonders bevorzugt 0,01-2,5 Gew.-% eines oder mehrerer Vernetzer,
(α4) 0 bis 5 Gew.-%, vorzugsweise 0,001 bis 2,5 Gew.-% und am meisten bevorzugt 0,01 bis 1 Gew.% des Metallsalzes,
(α5) 0 bis 5 Gew.-%, vorzugsweise 0,01 bis 2,5 Gew.-% und am meisten bevorzugt 0,1 bis 1 Gew.-% des Oxides des Metalls,
(α6) 0 bis 30 Gew.-%, vorzugweise 0 bis 5 Gew.-% und besonders bevorzugt 0.1-5 Gew.-% eines wasserlöslichen Polymeren,
(α7) 0 bis 20 vorzugsweise 2.5 bis 15 Gew.-% und besonders bevorzugt 3 bis 10 Gew.-% Wasser, sowie (α8) 0 bis 20 Gew.%, vorzugsweise 0 bis 10 Gew.-% und besonders bevorzugt 0,1 bis 8 Gew. -% eines oder mehrerer Hilfsmittel, wobei die Summe der Gewichtsmengen (α1) bis (α8) 100 Gew.% bestehen;
ii. einen Geruchsbinder beinhaltend als Komponenten
b1. eine Aminosäure; und
b2. eine Verbindung der allgemeinen Formel I 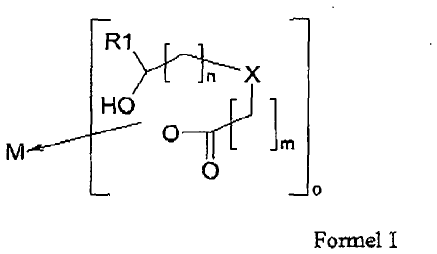 mit
R1 als einen C₁- bis C₂₀-Kohlenwasserstoff;
X als nicht vorhanden oder ein in einem Bereich von 1 bis 5 Doppelbindungen aufweisendes, im Falle von mindestens 2 Doppelbindungen vorzugsweise konjugiertes Doppelbindungssystem;
M als ein geladenes oder ungeladenes Metall;
m in einem Bereich von 1 bis 15;
n in einem Bereich von 1 bis 5;
o in einem Bereich von 1 bis 4,
erhältlich durch Inkontaktbringen der Oberfläche des wasserabsorbierenden Polymergebildes mit dem Geruchsbinder.

2. Zusammensetzung nach Anspruch 1, wobei das Metall M ausgewählt ist aus der Gruppe bestehend aus einwertigen, zweiwertigen oder dreiwertigen Metallen.

3. Zusammensetzung nach einem der vorhergehenden Absprüche, wobei die Komponente b2 ein Metallsalz der Ricinolsäure ist.

4. Zusammensetzung nach einem der vorhergehenden Ansprüche, wobei die Komponente b2 das Zu-Salz der Ricinolsäure ist.

5. Zusammensetzung nach einem der vorhergehenden Ansprüche, wobei das wasserabsorbierende Polymergebilde auf weniger als 40 Gew.-%, bezogen auf das wasserabsorbierende Polymergebilde, Superabsorberfeinteilchen einer Partikelgröße von weniger als 150 µm, bestimmt nach ERT 420.1-99, basiert.

6. Zusammensetzung nach einem der vorhergehenden Ansprüche, wobei das wasserabsorbierende Polymergebilde mindestens 60 Gew.%, bezogen auf das wasserabsorbierende Polymergebilde, Teilchen mit einer Teilchengröße in einem Bereich von 150 bis 850 µm, bestimmt nach ERT 420.1-99, aufweist.

7. Zusammensetzung nach einem der vorhergehenden Ansprüche, wobei diese auf weniger als 20 Gew.-%, bezogen auf die Zusammensetzung, eines vernetzbaren, nicht vernetzten Polymers basiert.

8. Zusammensetzung nach einem der vorhergehenden Ansprüche, wobei das Polymergebilde
- einen Innenbereich, sowie
- einen den Innenbereich umgebenden Außenbereich aufweist und wobei der Außenbereich einen höheren Vernetzungsgrad aufweist als der Innenbereich.

9. Zusammensetzung nach Anspruch 8, wobei die Konzentration des Geruchsbinders in oder auf dem Außenbereich höher ist als die Konzentration des Geruchsbinders in dem Innenbereich.

10. Zusammensetzung nach einem der vorhergehenden Ansprüchen, wobei das Polymergebilde
- einen Innenbereich, sowie
- einen den Innenbereich umgebenden Außenbereich aufweist und wobei die Konzentration des Geruchsbinders in oder auf dem Außenbereich höher ist als die Konzentration des Geruchsbinders in dem Innenbereich.

11. Zusammensetzung nach einem der vorhergehenden Ansprüche, wobei das wasserabsorbierende Polymergebilde mit einer von Komponente b1 verschiedenen Verbindung oberflächennachvernetzt ist.

12. Zusammensetzung nach einem der vorhergehenden Ansprüche, wobei die Aminosäure eine α-, β- oder γ-Aminosäure ist.

13. Zusammensetzung nach einem der vorhergehenden Ansprüche, wobei die Aminosäure Cystein ist.

14. Zusammensetzung nach einem der vorhergehenden Ansprüche, weiterhin beinhaltend iii. einen von dem wasserabsorbierenden Polymergebilde verschiedenen polymeren Binder.

15. Zusammensetzung nach Anspruch 14, wobei der polymere Binder zwei oder mehr OH-Gruppen beinhaltet.

16. Verfahren zur Herstellung einer superabsorbierenden Zusammensetzung, beinhaltend die Verfahrensschritte:
I. Bereitstellen eines wasserabsorbierenden Polymergebildes, worin das eingesetzte wasserabsorbierende Polymer gebilde aus
(α1) 20-99,999 Gew.-%, bevorzugt 55-98,99 Gew.-% und besonders bevorzugt 70-98,79 Gew.-% polymerisierten, ethylenisch ungesättigten, säuregruppen-tragenden Monomeren oder deren Salze oder polymerisierten, ethylenisch ungesättigten, einen protonierten oder quarternierten Stickstoff beinhaltenden Monomeren, oder deren Mischungen, wobei mindestens ethylenisch ungesättigte, säuregruppenhaltige Monomere, vorzugsweise Acrylsäure, beinhaltende Mischungen besonders bevorzugt sind,
(α2) 0-80 Gew.-%, vorzugsweise 0-44,99 Gew.-% und besonders bevorzugt 0,1-44,89 Gew.-% polymerisierten, monoethylenisch ungesättigten, mit (α1) copolymerisierbaren Monomeren,
(α3) 0,001-5 Gew.-%, vorzugsweise 0.01-3 Gew.-% und besonders bevorzugt 0,01-2,5 Gew.% eines oder mehrerer Vernetzer,
(α4) 0 bis 5 Gew.-%, vorzugsweise 0.001 bis 2,5 Gew.-% und am meisten bevorzugt 0.01 bis 1 Gew.-% des Metallsalzes,
(α5) 0 bis 5 Gew.-%, vorzugsweise 0,01 bis 2,5 Gew.% und am meisten bevorzugt 0.1 bis 1 Gew.-% des Oxides des Metalls,
(α6) 0 bis 30 Gew.%, vorzugsweise 0 bis 5 Gew.-% und besonders bevorzugt 0.1-5 Gew.-% eines wasserlöslichen Polymeren,
(α7) 0 bis 20 Gew.%, vorzugsweise 2,5 bis 15 Gew.-% und besonders bevorzugt 3 bis 10 Gew.-% Wasser, sowie (α8) 0 bis 20 Gew.-% vorzugsweise 0 bis 10 Gew.-% und besonders bevorzugt 0,1 bis 8 Gew.-% eines oder mehrerer Hilfsmittel, wobei die Summe der Gewichtsmengen (α1) bis (α8) 100 Gew. -% bestehen:
II. Inkontaktbringen der Oberfläche des wasserabsorbierenden Polymergebildes mit einem Geruchsbinder beinhaltend als Komponenten
B1. eine Aminosäure; und
B2. eine Verbindung der allgemeinen Fromel I 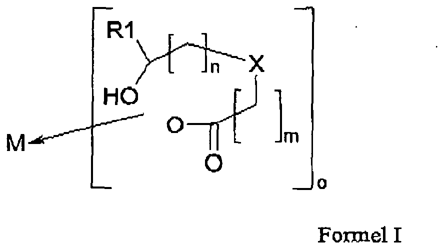 mit
R1 als einen C₁- bis C₂₀-Kohlenwasserstoff;
X als nicht vorhanden oder ein in einem Bereich von 1 bis 5 Doppelbindungen aufweisendes, im Falle von mindestens 2 Doppelbindungen vorzugsweise konjugiertes Doppelbindungssystem;
M als ein geladenes oder ungeladenes Metall;
m in einem Bereich von 1 bis 15;
n in einem Bereich von 1 bis 5;
o in einem Bereich von 1 bis 4.

17. Verfahren nach Anspruch 16, wobei das Metall M ausgewählt ist aus der Gruppe bestehend aus einwertigen, zweiwertigen oder dreiwertigen Metallen.

18. Verfahren nach einem der Ansprüche 16 oder 17, wobei die Komponente B2 ein Metallsalz der Ricinolsäure ist.

19. Verfahren nach einem der Ansprüche 16 bis 18, wobei die Komponente B2 ein Zn-Salz der Ricinolsäure ist.

20. Verfahren nach einem der Ansprüche 16 bis 19, wobei das wasserabsorbierende Polymergebilde als Hydrogel vorliegt.

21. Verfahren nach einem der Ansprüche 16 bis 20, wobei das wasserabsorbierende Polymergebilde einen Wassergehalt von weniger als 10 Gew.-%, bezogen auf das Polymergebilde, hat.

22. Verfahren nach einem der Ansprüche 16 bis 21, wobei das Inkontaktbringen weiterhin in Gegenwart eines polymeren Binders III. erfolgt.

23. Verfahren nach einem der Ansprüche 16 bis 22, wobei das wasserabsorbierende Polymergebilde vor dem Inkontaktbringen oberflächenvernetzt wird.

24. Verfahren nach einem der Ansprüche 16 bis 23, wobei das wasserabsorbierende Polymergebilde auf weniger als 40 Gew.-%, bezogen auf das wasserabsorbierende Polymergebildes, Superabsorberfeinteilchen einer Partikelgröße von weniger als 150 µm, bestimmt nach ERT 420.1-99, basiert.

25. Verfahren nach einem der Ansprüche 16 bis 24, wobei das wasserabsorbierende Polymergebilde mindestens 60 Gew.-%, bezogen auf das wasserabsorbierende Polymergebilde, Teilchen mit einer Teilchengröße in einem Bereich von 150 bis 850 µm, bestimmt nach ERT 420.1-99, aufweist.

26. Verfahren nach einem der Ansprüche 16 bis 25, wobei weniger als 20 Gew.-%, bezogen auf die Zusammensetzung, eines vernetzbaren, nicht vernetzten Polymers als ein Binder eingesetzt wird.

27. Verbund, beinhaltend eine superabsorbierenden Zusammensetzung nach einen der Ansprüche 1 bis 15 oder erhältlich nach einem Verfahren nach eines der Ansprüche 16 bis 26 und ein Substrat.

28. Verbund nach Anspruch 27 als Hygineartikelcore ausgebildet, beinhaltend, jeweils bezogen auf das Hygieneartikelcore, mindestens 30 Gew.-% der superabsorbierenden Zusammensetzung nach einem der Ansprüche 1 bis 15 oder erhältlich nach einem Verfahren nach einem der Ansprüche 16 bis 26 und mindestens 1 Gew.-% des Substrats.

29. Hygieneartikel, beinhaltend eine flüssigkeitsdurchlässige Oberschicht, eine flüssigkeitsundurchlässige Unterschicht und einen zwischen der Oberschicht und der Unterschicht angeordneten Verbund nach einem der Ansprüche 27 oder 28.

30. Schäume, Formkörper, Fasern, Folien, Filme, Kabel, Dichtungsmaterialien, flüssigkeitsaufnehmende Hygieneartikel, Träger für pflanzen- und pilzwachstmnregulierende Mittel, Verpackungsmaterialien, Bodenzusätze oder Baustoffe, beinhaltend eine superabsorbierende Zusammensetzung nach einem der Ansprüche 1 bis 15 oder erhältlich nach einem Verfahren nach einem der Ansprüche 16 bis 26 oder einen Verbund nach einem der Ansprüche 27 oder 28.

31. Verwendung einer superabsorbierenden Zusammensetzung nach einem der Ansprüche 1 bis 15 oder erhältlich nach einem Verfahren nach einem der Ansprüche 16 bis 26 oder eines Verbunds nach einem der Ansprüche 27 oder 28 in Schäumen, Formkörpern, Fasern, Folien, Filmen, Kabeln, Dichtungsmaterialien, flüssigkeitsaufnehmenden Hygieneartikeln, Trägem für pflanzen- und pilzwachstumregulierenden Mitteln, Verpackungsmaterialien, Bodenzusätzen, zur kontrollierten Freisetzung von Wirkstoffen oder in Baustoffen.

## Claims

1. Odour-absorbent superabsorbent composition comprising
i. a water-absorbing polymer structure, in which the water-absorbing polymer structure used consists of
(α1) 20-99.999% by weight, preferably 55-98.99% by weight and more preferably 70-98.79% by weight of polymerized, ethylenically unsaturated monomers bearing acid groups or salts thereof or polymerized, ethylenically unsaturated monomers including a protonated or quaternized nitrogen, or mixtures thereof, particular preference being given to mixtures including at least ethylenically unsaturated monomers bearing acid groups, preferably acrylic acid,
(α2) 0-80% by weight, preferably 0-44.99% by weight and more preferably 0.1-44.89% by weight of polymerized, monoethylenically unsaturated monomers copolymerizable with (α1),
(α3) 0.001-5% by weight, preferably 0.01-3% by weight and more preferably 0.01-2.5% by weight of one or more crosslinkers,
(α4) 0 to 5% by weight, preferably 0.001 to 2.5% by weight and most preferably 0.01 to 1% by weight of the metal salt,
(α5) 0 to 5% by weight, preferably 0.01 to 2.5% by weight and most preferably 0.1 to 1% by weight of the oxide of the metal,
(α6) 0 to 30% by weight, preferably 0 to 5% by weight and more preferably 0.1-5% by weight of a water-soluble polymer,
(α7) 0 to 20% by weight, preferably 2.5 to 15% by weight and more preferably 3 to 10% by weight of water, and
(α8) 0 to 20% by weight, preferably 0 to 10% by weight and more preferably 0.1 to 8% by weight of one or more assistants, where the sum of the weights (α1) to (α8) is 100% by weight;
ii. an odour binder comprising as components
b1. an amino acid; and
b2. a compound of the general formula I 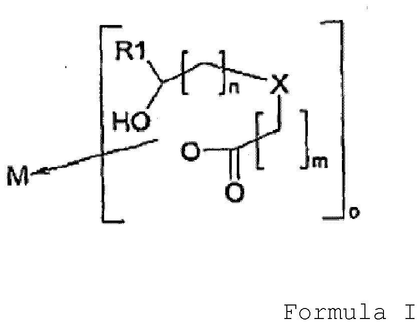 where
R1 is a C₁ to C₂₀ hydrocarbon;
X is absent or is a double bond system which has within a range from 1 to 5 double bonds and is preferably conjugated in the case of at least 2 double bonds;
M is a charged or uncharged metal;
m is within a range from 1 to 15;
n is within a range from 1 to 5;
o is within a range from 1 to 4,
obtainable by contacting the surface of the water-absorbing polymer structure with the odour binder.

2. Composition according to Claim 1, wherein the metal M is selected from the group consisting of monovalent, divalent or trivalent metals.

3. Composition according to either of the preceding claims, wherein component b2 is a metal salt of ricinoleic acid.

4. Composition according to any of the preceding claims, wherein component b2 is the zinc salt of ricinoleic acid.

5. Composition according to any of the preceding claims, wherein the water-absorbing polymer structure is based on less than 40% by weight, based on the water-absorbing polymer structure, of fine superabsorbent particles of particle size less than 150 µm, determined to ERT 420.1-99.

6. Composition according to any of the preceding claims, wherein the water-absorbing polymer structure has at least 60% by weight, based on the water-absorbing polymer structure, of particles having a particle size within a range from 150 to 850 µm, determined to ERT 420.1-99.

7. Composition according to any of the preceding claims, which is based on less than 20% by weight, based on the composition, of a crosslinkable, uncrosslinked polymer.

8. Composition according to any of the preceding claims, wherein the polymer structure has
- an inner region and
- an outer region surrounding the inner region, and wherein the outer region has a higher degree of crosslinking than the inner region.

9. Composition according to Claim 8, wherein the composition of the odour binder in or on the outer region is higher than the concentration of the odour binder in the inner region.

10. Composition according to any of the preceding claims, wherein the polymer structure has
- an inner region and
- an outer region surrounding the inner region, and wherein the concentration of the odour binder in or on the outer region is higher than the concentration of the odour binder in the inner region.

11. Composition according to any of the preceding claims, wherein the water-absorbing polymer structure has been surface postcrosslinked with a compound other than component b1.

12. Composition according to any of the preceding claims, wherein the amino acid is an α-, β- or γ-amino acid.

13. Composition according to any of the preceding claims, wherein the amino acid is cysteine.

14. Composition according to any of the preceding claims, further comprising iii. a polymeric binder other than the water-absorbing polymer structure.

15. Composition according to Claim 14, wherein the polymeric binder comprises two or more OH groups.

16. Process for producing a superabsorbent composition, comprising the process steps of:
I. providing a water-absorbing polymer structure, in which the water-absorbing polymer structure used consists of
(α1) 20-99.999% by weight, preferably 55-98.99% by weight and more preferably 70-98.79% by weight of polymerized, ethylenically unsaturated monomers bearing acid groups or salts thereof or polymerized, ethylenically unsaturated monomers including a protonated or quaternized nitrogen, or mixtures thereof, particular preference being given to mixtures including at least ethylenically unsaturated monomers bearing acid groups, preferably acrylic acid,
(α2) 0-80% by weight, preferably 0-44.99% by weight and more preferably 0.1-44.89% by weight of polymerized, monoethylenically unsaturated monomers copolymerizable with (α1),
(α3) 0.001-5% by weight, preferably 0.01-3% by weight and more preferably 0.01-2.5% by weight of one or more crosslinkers,
(α4) 0 to 5% by weight, preferably 0.001 to 2.5% by weight and most preferably 0.01 to 1% by weight of the metal salt,
(α5) 0 to 5% by weight, preferably 0.01 to 2.5% by weight and most preferably 0.1 to 1% by weight of the oxide of the metal,
(α6) 0 to 30% by weight, preferably 0 to 5% by weight and more preferably 0.1-5% by weight of a water-soluble polymer,
(α7) 0 to 20% by weight, preferably 2.5 to 15% by weight and more preferably 3 to 10% by weight of water, and
(α8) 0 to 20% by weight, preferably 0 to 10% by weight and more preferably 0.1 to 8% by weight of one or more assistants, where the sum of the weights (α1) to (α8) is 100% by weight;
II. contacting the surface of the water-absorbing polymer structure with an odour binder comprising as components
B1. an amino acid; and
B2. a compound of the general formula I 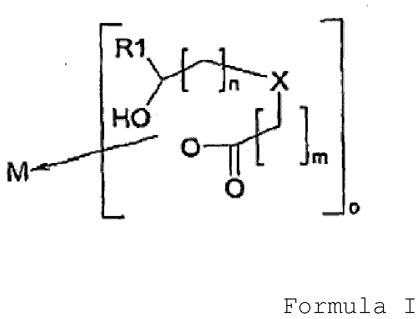 where
R1 is a C₁ to C₂₀ hydrocarbon;
X is absent or is a double bond system which has within a range from 1 to 5 double bonds and is preferably conjugated in the case of at least 2 double bonds;
M is a charged or uncharged metal;
m is within a range from 1 to 15;
n is within a range from 1 to 5;
o is within a range from 1 to 4.

17. Process according to Claim 16, wherein the metal M is selected from the group consisting of monovalent, divalent or trivalent metals.

18. Process according to either of Claims 16 and 17, wherein component B2 is a metal salt of ricinoleic acid.

19. Process according to any of Claims 16 to 18, wherein component B2 is a zinc salt of ricinoleic acid.

20. Process according to any of Claims 16 to 19, wherein the water-absorbing polymer structure is in the form of a hydrogel.

21. Process according to any of Claims 16 to 20, wherein the water-absorbing polymer structure has a water content of less than 10% by weight, based on the polymer structure.

22. Process according to any of Claims 16 to 21, wherein the contacting is also effected in the presence of a polymeric binder III.

23. Process according to any of Claims 16 to 22, wherein the water-absorbing polymer structure is surface crosslinked prior to contacting.

24. Process according to any of Claims 16 to 23, wherein the water-absorbing polymer structure is based on less than 40% by weight, based on the water-absorbing polymer structure, of fine superabsorbent particles of particle size less than 150 µm, determined to ERT 420.1-99.

25. Process according to any of Claims 16 to 24, wherein the water-absorbing polymer structure has at least 60% by weight, based on the water-absorbing polymer structure, of particles having a particle size within a range from 150 to 850 µm, determined to ERT 420.1-99.

26. Process according to any of Claims 16 to 25, wherein less than 20% by weight, based on the composition, of a crosslinkable, uncrosslinked polymer is used as a binder.

27. Composite comprising a superabsorbent composition according to any of Claims 1 to 15 or obtainable by a process according to any of Claims 16 to 26 and a substrate.

28. Composite according to Claim 27 in the form of a hygiene article core, comprising, based in each case on the hygiene article core, at least 30% by weight of the superabsorbent composition according to any of Claims 1 to 15 or obtainable by a process according to any of Claims 16 to 26 and at least 1% by weight of the substrate.

29. A hygiene article comprising a liquid-pervious top layer, a liquid-impervious bottom layer and a composite according to either of Claims 27 and 28 arranged between the top layer and the bottom layer.

30. Foams, mouldings, fibres, foils, films, cables, sealing materials, liquid-absorbing hygiene articles, carriers for plant growth and fungal growth regulators, packaging materials, soil additives or building materials comprising a superabsorbent composition according to any of Claims 1 to 15 or obtainable by a process according to any of Claims 16 to 26 or a composite according to either of Claims 27 and 28.

31. The use of a superabsorbent composition according to any of Claims 1 to 15 or obtainable by a process according to any of Claims 16 to 26 or of a composite according to either of Claims 27 and 28 in foams, mouldings, fibres, foils, films, cables, sealing materials, liquid-absorbing hygiene articles, carriers for plant growth and fungal growth regulators, packaging materials, soil additives, for controlled release of active ingredients, or in building materials.

## Revendications

1. Composition superabsorbante absorbant les odeurs, comportant
i. une structure polymère absorbant l'eau, la structure polymère absorbant l'eau, qui est utilisée, étant constituée de
(α1) 20-99,999 % en poids, de préférence 55-98,99 % en poids et de façon particulièrement préférée 70-98,79 % en poids de monomères à insaturation éthylénique portant des groupes acides ou de leurs sels, polymérisés, ou de monomères à insaturation éthylénique comportant un atome d'azote protoné ou rendu quaternaire, polymérisés, ou de mélanges de ceux-ci, les mélanges comportant au moins des monomères à insaturation éthylénique portant des groupes acide, l'acide acrylique de préférence, étant particulièrement préférés,
(α2) 0-80 % en poids, de préférence 0-44,99 % en poids et de façon particulièrement préférée 0,1-44,89 % en poids de monomères à insaturation monoéthylénique, copolymérisables avec (α1), polymérisés,
(α3) 0,001-5 % en poids, de préférence 0,01-3 % en poids et de façon particulièrement préférée 0,01-2,5 % en poids d'un ou plusieurs agents de réticulation,
(α4) 0 à 5 % en poids, de préférence 0,001 à 2,5 % en poids et de façon tout particulièrement préférée 0,01 à 1 % en poids du sel métallique, (α5) 0 à 5 % en poids, de préférence 0,01 à 2,5 % en poids et de façon tout particulièrement préférée 0,1 à 1 % en poids de l'oxyde du métal, (α6) 0 à 30 % en poids, de préférence 0 à 5 % en poids et de façon particulièrement préférée 0,1-5 % en poids d'un polymère hydrosoluble,
(α7) 0 à 20 % en poids, de préférence 2,5 à 15 % en poids et de façon particulièrement préférée 3 à 10 % en poids d'eau, ainsi que
(α8) 0 à 20 % en poids, de préférence 0 à 10 % en poids et de façon particulièrement préférée 0,1 à 8 % en poids d'un ou plusieurs adjuvants, la somme des quantités en poids de (α1) à (α8) étant égale à 100 % en poids ;
ii. un capteur d'odeurs, comportant comme composants
b1. un acide aminé ; et
b2. un composé de formule générale I 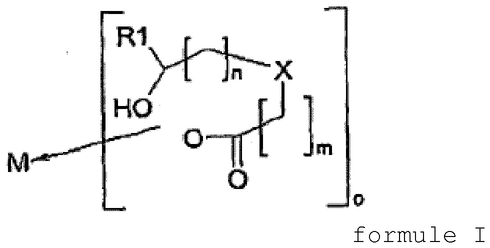
où
R1 représente un hydrocarbure en C₁-C₂₀ ;
X est absent ou représente un système de double(s) liaison(s) comportant dans une plage de 1 à 5 doubles liaisons, dans le cas d'au moins 2 doubles liaisons de préférence conjugué ;
M représente un métal chargé ou non chargé ;
m a une valeur dans une plage de 1 à 15 ;
n a une valeur dans une plage de 1 à 5 ;
o a une valeur dans une plage de 1 à 4,
pouvant être obtenue par mise en contact de la surface de la structure polymère absorbant l'eau avec le capteur d'odeurs.

2. Composition selon la revendication 1, dans laquelle le métal M est choisi dans le groupe constitué par des métaux monovalents, divalents ou trivalents.

3. Composition selon l'une quelconque des revendications précédentes, dans laquelle le composant b2 est un sel métallique de l'acide ricinoléique.

4. Composition selon l'une quelconque des revendications précédentes, dans laquelle le composant b2 est le sel de Zn de l'acide ricinoléique.

5. Composition selon l'une quelconque des revendications précédentes, dans laquelle la structure polymère absorbant l'eau est à base de moins de 40 % en poids, par rapport à la structure polymère absorbant l'eau, de particules de superabsorbant ayant une taille de particule de moins de 150 µm, déterminée selon ERT 420.1-99.

6. Composition selon l'une quelconque des revendications précédentes, dans laquelle la structure polymère absorbant l'eau est à base d'au moins 60 % en poids, par rapport à la structure polymère absorbant l'eau, de particules ayant une taille de particule dans une plage de 150 à 850 µm, déterminée selon ERT 420.1-99.

7. Composition selon l'une quelconque des revendications précédentes, celle-ci étant à base de moins de 20 % en poids, par rapport à la composition, d'un polymère réticulable, non réticulé.

8. Composition selon l'une quelconque des revendications précédentes, dans laquelle la structure polymère comporte
- une zone interne, ainsi
- qu'une zone externe entourant la zone interne et dans laquelle la zone externe présente un plus haut degré de réticulation que la zone interne.

9. Composition selon la revendication 8, dans laquelle la concentration du capteur d'odeurs dans ou sur la zone externe est supérieures à la concentration du capteur d'odeurs dans la zone interne.

10. Composition selon l'une quelconque des revendications précédentes, dans laquelle la structure polymère comporte
- une zone interne, ainsi
- qu'une zone externe entourant la zone interne et dans laquelle la concentration du capteur d'odeurs dans ou sur la zone externe est supérieure à la concentration du capteur d'odeurs dans la zone interne.

11. Composition selon l'une quelconque des revendications précédentes, dans laquelle la structure polymère absorbant l'eau est réticulée en surface avec un composé différent du composé b1.

12. Composition selon l'une quelconque des revendications précédentes, dans laquelle l'acide aminé est une acide α-, β- ou γ-aminé.

13. Composition selon l'une quelconque des revendications précédentes, dans laquelle l'acide aminé est la cystéine.

14. Composition selon l'une quelconque des revendications précédentes, en outre comportant iii. un liant polymère différent de la structure polymère absorbant l'eau.

15. Composition selon la revendication 14, dans laquelle le liant polymère comporte deux ou plus de deux groupes OH.

16. Procédé pour la préparation d'une composition superabsorbante, comportant les étapes de processus :
I. fourniture d'une structure polymère absorbant l'eau, la structure polymère absorbant l'eau qui est utilisée étant constituée de
(α1) 20-99,999 % en poids, de préférence 55-98,99 % en poids et de façon particulièrement préférée 70-98,79 % en poids de monomères à insaturation éthylénique portant des groupes acides ou de leurs sels, polymérisés, ou de monomères à insaturation éthylénique comportant un atome d'azote protoné ou rendu quaternaire, polymérisés, ou de mélanges de ceux-ci, des mélanges comportant au moins des monomères à insaturation éthylénique portant des groupes acide, l'acide acrylique de préférence, étant particulièrement préférés,
(α2) 0-80 % en poids, de préférence 0-44,99 % en poids et de façon particulièrement préférée 0,1-44,89 % en poids de monomères à insaturation monoéthylénique, copolymérisables avec (α1), polymérisés,
(α3) 0,001-5 % en poids, de préférence 0,01-3 % en poids et de façon particulièrement préférée 0,01-2,5 % en poids d'un ou plusieurs agents de réticulation,
(α4) 0 à 5 % en poids, de préférence 0,001 à 2,5 % en poids et de façon tout particulièrement préférée 0,01 à 1 % en poids du sel métallique,
(α5) 0 à 5 % en poids, de préférence 0,01 à 2,5 % en poids et de façon tout particulièrement préférée 0,1 à 1 % en poids de l'oxyde du métal,
(α6) 0 à 30 % en poids, de préférence 0 à 5 % en poids et de façon particulièrement préférée 0,1-5 % en poids d'un polymère hydrosoluble,
(α7) 0 à 20 % en poids, de préférence 2,5 à 15 % en poids et de façon particulièrement préférée 3 à 10 % en poids d'eau, ainsi que
(α8) 0 à 20 % en poids, de préférence 0 à 10 % en poids et de façon particulièrement préférée 0,1 à 8 % en poids d'un ou plusieurs adjuvants, la somme des quantités en poids de (α1) à (α8) étant égale à 100 % en poids ;
II. mise en contact de la surface de la structure polymère absorbant l'eau avec un capteur d'odeurs, comportant comme composants
B1. un acide aminé ; et
B2. un composé de formule générale I 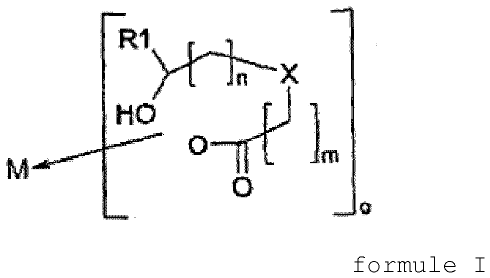
où
R1 représente un hydrocarbure en C₁-C₂₀ ;
X est absent ou représente un système de double(s) liaison(s) comportant dans une plage de 1 à 5 doubles liaisons, dans le cas d'au moins 2 doubles liaisons de préférence conjugué ;
M représente un métal chargé ou non chargé ;
m a une valeur dans une plage de 1 à 15 ;
n a une valeur dans une plage de 1 à 5 ;
o a une valeur dans une plage de 1 à 4.

17. Procédé selon la revendication 16, dans lequel le métal M est choisi dans le groupe constitué par des métaux monovalents, divalents ou trivalents.

18. Procédé selon la revendication 16 ou 17, dans lequel le composant B2 est un sel métallique de l'acide ricinoléique.

19. Procédé selon l'une quelconque des revendications 16 à 18, dans lequel le composant B2 est un sel de Zn de l'acide ricinoléique.

20. Procédé selon l'une quelconque des revendications 16 à 19, dans lequel la structure polymère absorbant l'eau est présente sous forme d'hydrogel.

21. Procédé selon l'une quelconque des revendications 16 à 20, dans lequel la structure polymère absorbant l'eau a une teneur en eau de moins de 10 % en poids, par rapport à la structure polymère absorbant l'eau.

22. Procédé selon l'une quelconque des revendications 16 à 21, dans lequel la mise en contact s'effectue de surcroît en présence d'un liant polymère III.

23. Procédé selon l'une quelconque des revendications 16 à 22, dans lequel avant la mise en contact on soumet à une réticulation superficielle la structure polymère absorbant l'eau.

24. Procédé selon l'une quelconque des revendications 16 à 23, dans lequel la structure polymère absorbant l'eau est à base de moins de 40 % en poids, par rapport à la structure polymère absorbant l'eau, de particules de superabsorbant ayant une taille de particule de moins de 150 µm, déterminée selon ERT 420.1-99.

25. Procédé selon l'une quelconque des revendications 16 à 24, dans lequel la structure polymère absorbant l'eau comporte au moins 60 % en poids, par rapport à la structure polymère absorbant l'eau, de particules ayant une taille de particule dans une plage de 150 à 850 µm, déterminée selon ERT 420.1-99.

26. Procédé selon l'une quelconque des revendications 16 à 25, dans lequel on utilise comme liant moins de 20 % en poids, par rapport à la composition, d'un polymère réticulable, non réticulé.

27. Composite, comportant une composition superabsorbante selon l'une quelconque des revendications 1 à 15 ou pouvant être obtenue conformément à un procédé selon l'une quelconque des revendications 16 à 26 et un support.

28. Composite selon la revendication 27, conçu en tant qu'âme d'article d'hygiène, comportant, chaque fois par rapport à l'âme de l'article d'hygiène, au moins 30 % en poids de la composition superabsorbante selon l'une quelconque des revendications 1 à 15 ou pouvant être obtenue conformément à un procédé selon l'une quelconque des revendications 16 à 26 et au moins 1 % en poids du support.

29. Article d'hygiène, comportant une couche supérieure perméable aux liquides, une couche inférieure imperméable aux liquides et un composite selon la revendication 27 ou 28, disposé entre la couche supérieure et la couche inférieure.

30. Mousses, corps moulés, fibres, feuilles, films, câbles, matériaux d'étanchéité, articles d'hygiène absorbant les liquides, supports pour agents régulateurs de croissance de plantes et de champignons, matériaux d'emballage, additifs pour sols ou matériaux de construction, comportant une composition superabsorbante selon l'une quelconque des revendications 1 à 15 ou pouvant être obtenue conformément à un procédé selon l'une quelconque des revendications 16 à 26 ou un composite selon la revendication 27 ou 28.

31. Utilisation d'une composition superabsorbante selon l'une quelconque des revendications 1 à 15 ou pouvant être obtenue conformément à un procédé selon l'une quelconque des revendications 16 à 26 ou d'un composite selon la revendication 27 ou 28, dans des mousses, corps moulés, fibres, feuilles, films, câbles, matériaux d'étanchéité, articles d'hygiène absorbant les liquides, supports pour agents régulateurs de croissance de plantes et de champignons, matériaux d'emballage, additifs pour sols, pour la libération programmée de substances actives ou dans des matériaux de construction.
